# NEUE EUROPÄISCHE PATENTSCHRIFT

(11) **EP 2 644 218 B2**
(45) Veröffentlichungstag und Bekanntmachung des Hinweises auf die Entscheidung über den Einspruch: **02.11.2022**
(45) Hinweis auf die Patenterteilung: 09.10.2019
(21) Anmeldenummer: 12178912.7
(22) Anmeldetag: 01.08.2012
(51) Int. Cl.: A61M 5/20, A61M 5/24, A61M 5/31, A61M 5/315

(54) **Injektionsvorrichtung mit Dosisanzeige und Federantrieb**
Injection device with dose display and clockwork drive
Dispositif d'injection avec affichage de dose et entraînement par ressort

(30) Priorität: 30.03.2012 EP 12162777
(43) Veröffentlichungstag der Anmeldung: 02.10.2013
(73) Patentinhaber: Tecpharma Licensing AG, 3401 Burgdorf (CH)
(72) Erfinder: Streit, Ursina, 3322 Schönbühl (CH); Schenker, Susanne, 4900 Langenthal (CH)
(74) Vertreter: Meier Obertüfer, Jürg

(56) Entgegenhaltungen:
- WO-A1-2008/087071
- WO-A1-2010/081489
- WO-A1-2010/115670
- WO-A1-2012/037938

## Beschreibung

Die Erfindung betrifft eine Injektionsvorrichtung zum Verabreichen eines flüssigen Produkts, insbesondere eines Medikaments, wie z. B. Insulin zur Diabetestherapie. Im Besonderen betrifft die Erfindung eine Antriebs- und Dosiervorrichtung für eine solche Injektionsvorrichtung.

Aus dem Stand der Technik, nämlich der WO 2008/031237 A1 ist ein Injektionsgerät mit einer Dosisanzeigetrommel und einer Antriebsfeder bekannt. Die Antriebsfeder ist eine Uhrenfeder, die spiralförmig aus einem bandförmigen Material gewickelt ist. Beim Einstellen der gewünschten Produktdosis wird die Feder mit einer rotatorischen Bewegung gespannt. Für die Dosisausschüttung wird durch Betätigen eines Betätigungsknopfs am proximalen Ende der Vorrichtung eine Kolbenstange der Vorrichtung mit der Feder gekoppelt, wodurch die Feder die in ihr gespeicherte Energie an die Kolbenstange abgeben kann, wodurch die Kolbenstange in Ausschüttrichtung bewegt wird. Zum Einstellen einer neuen Dosis wird die Feder durch Drehen des Dosierknopfs wieder vorgespannt und so weiter. Dies wird so oft wiederholt, bis der Produktbehälter geleert ist. Aus der WO2012/037938 ist ebenso eine Injektionsvorrichtung bekannt.

Aus der US 5,104,380 A ist eine Injektionsvorrichtung mit einer Schraubenfeder bekannt, welche bei der Dosierung ebenfalls rotatorisch vorgespannt wird, so dass die Schraubenfeder als Torsionsfeder bezeichnet werden kann. Die vor jeder Produktausschüttung durch Drehen eines Drehknopfs vorgespannte Feder gibt ihre Energie zum Vortrieb der Kolbenstange an die Kolbenstange ab.

Aus der WO 2006/77466 A2 ist eine Injektionsvorrichtung bekannt, die einen direkten mechanischen Antrieb zwischen der Person, welche die Injektionskraft aufbringt, und der Kolbenstange, die für die Injektion des Medikaments in distale Richtung verschoben wird, aufweist.

Es ist eine Aufgabe der Erfindung eine Antriebs- und Dosiervorrichtung für eine Injektionsvorrichtung mit einer verbesserten Dosisanzeige anzugeben und die dem Verwender der Vorrichtung insbesondere verbessert Aufschluss über den Betriebszustand der Vorrichtung gibt.

Die Aufgabe wird durch die Merkmale des Anspruchs 1 gelöst. Vorteilhafte Weiterbildungen ergeben sich aus den abhängigen Ansprüchen, der Beschreibung und den Figuren.

Die Erfindung geht von einem Antriebsmechanismus für eine Injektionsvorrichtung zum Verabreichen eines flüssigen Medikaments oder Produkts aus. Der Antriebsmechanismus weist ein Gehäuse auf. Das Gehäuse ist vorzugsweise hülsenförmig und/oder länglich ausgebildet. Das Gehäuse kann sich z. B. entlang seiner Längsachse erstrecken.

Das Gehäuse kann optional einen Produktbehälter aufnehmen oder selbst den Produktbehälter bilden. Das Gehäuse kann ein- oder mehrteilig sein. Z. B. kann das Gehäuse einen proximalen Gehäuseteil bilden, der die Antriebs- und Dosiervorrichtung umfasst oder aufweist. Das Gehäuse kann ferner einen Produktbehälterhalter aufweisen, der den Produktbehälter, wie z. B. eine Karpule, aufnimmt und mit dem Gehäuse oder dem proximalen Gehäuseteil verbunden ist. Diese Verbindung kann derart sein, dass der Produktbehälterhalter und das Gehäuse oder der proximale Gehäuseteil nach dem Verbinden unlösbar, d. h. nur durch Zerstörung von Verbindungselementen lösbar ist. Eine solche Lösung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, wenngleich auch weniger bevorzugt, die Antriebs- und Dosiervorrichtung gegebenenfalls mehrfach zu verwenden, d. h. einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann z. B. eine Zeigeeinrichtung, insbesondere ein Fenster aufweisen, mittels der oder durch welches eine aktuell eingestellte Dosis, vorzugsweise von einer Skala eines Dosiseinstelleelements, ablesbar ist.

Die Antriebs- und Dosiervorrichtung, die insbesondere zusammen mit dem Produktbehälter eine Injektionsvorrichtung bildet, umfasst neben einem Gehäuse ein Dosisanzeigeelement, über dessen Umfang eine Dosisskala angeordnet ist. Das Dosisanzeigeelement kann z. B. im Querschnitt ringförmig sein. Das Dosisanzeigeelement kann z. B. eine Dosisanzeigetrommel oder ein Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Werten, die aneinandergereiht angeordnet sind und die Dosisskala ergeben. Vorzugsweise handelt es sich um Zahlenwerte, welche die gewünschte Produktdosis in internationalen Einheiten (IU) angeben.

Alternativ kann die Dosisskala ohne Steigung über den Umfang des Dosisanzeigeelements, wie z. B. des Dosisanzeigerings, angeordnet sein, wobei sich die Skalenwerte dann nach einer Umdrehung des Dosisanzeigeelements wiederholen. Bei einer Dosisskala mit Steigung, d. h. einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte dargestellt werden können.

Die Antriebs- und Dosiervorrichtung umfasst ferner eine Zeigeeinrichtung, wobei das Dosisanzeigeelement zur Einstellung der zu verabreichenden Dosis relativ zu der Zeigeeinrichtung und insbesondere um eine Drehachse, die vorzugsweise der Längsachse der Antriebs- und Dosiervorrichtung oder/und des Dosisanzeigeelements entspricht, drehbar ist. Hierbei kann es sich um eine reine Drehbewegung, d. h. eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Dosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegung und die Dosisskala vorteilhafterweise die gleiche Steigung aufweisen. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann z. B. ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der z. B. zusätzlich zu dem Fenster den Wert der Dosisskala markiert, welcher der eingestellten Dosis entspricht. Dies ist z. B. dann von Vorteil, wenn in dem Fenster noch ein anderer Wert zumindest teilweise erscheint, um eine eindeutige Dosisauswahl sicherzustellen. Der Zeiger kann z. B. ein Vorsprung oder ein Aufdruck oder eine Kerbe oder dergleichen sein.

Die Antriebs- und Dosiervorrichtung umfasst ein Dosierglied, das z.B. als Dosierknopf ausgebildet ist und optional als Dosiseinstellglied bezeichnet werden kann. Das Dosierglied ist vorzugsweise vom Verwender (Patient, Arzt, medizinisches Hilfspersonal) der Antriebs- und Dosiervorrichtung greibar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vorzugsweise vom Verwender ergriffen und relativ zu dem Gehäuse und insbesondere der Zeigeeinrichtung um eine Drehachse, die vorzugsweise der Längsachse der z.B. länglich ausgestalteten Antriebs- und Dosiervorrichtung entspricht, verdreht. Das Dosierglied ist vorzugsweise axialfest mit dem Gehäuse verbunden, insbesondere entlang einer Längsachse des Gehäuses verschiebefest, wodurch vorteilhaft die intuitive Handhabung der Vorrichtung durch den Verwender erleichtert wird, da er lediglich eine Drehbewegung des Dosierglieds zur Dosiseinstellung ausführen braucht.

Insbesondere kann das Dosisanzeigeelement zumindest während der Dosiseinstellung verdrehfest, aber z.B. axial verschiebbar mit dem Dosierglied verbunden oder gekoppelt sein.

Für die intuitive Bedienung ist es vorteilhaft, wenn das Dosisanzeigeelement bei Drehung des Dosierglieds um einen Drehwinkel ebenfalls um diesen Drehwinkel verdreht wird.

Die Antriebs- und Dosiervorrichtung kann ein Betätigungsglied z.B. in der Gestalt eines Betätigungsknopfs aufweisen. Das Betätigungsglied kann eine äußere Oberfläche der Antriebs- und Dosiervorrichtung bilden und/oder von außen zugänglich sein. Das Betätigungsglied kann am proximalen, insbesondere hinteren Ende der Antriebs- und Dosiervorrichtung gebildet sein oder dieses Ende bilden. Das Betätigungsglied lässt sich auf diese Weise vorteilhaft mit dem Daumen der Hand, die das Gehäuse umgreift betätigen, insbesondere drücken. Durch Loslassen des Betätigungsglieds kann die Betätigung beendet werden. Unter "Betätigen" wird die Verschiebung des Betätigungsglieds in die Antriebs- und Dosiervorrichtung, insbesondere in distale Richtung verstanden, wodurch insbesondere eine Produktausschüttung bewirkt werden kann. Das Betätigungsglied ist vorteilhaft relativ zu dem Dosierglied verschiebbar und kann insbesondere von dem Dosierglied axial verschiebbar aufgenommen sein.

Das Betätigungsglied kann vorteilhaft gegen die Kraft mindestens einer Feder, insbesondere einer Rücksetz- oder Kupplungsfeder verschiebbar, insbesondere betätigbar sein, wodurch diese mindestens eine Feder gespannt wird. Durch Loslassen kann diese Feder das Betätigungsglied zurücksetzen, insbesondere relativ zu dem Dosierglied, insbesondere in proximale Richtung oder aus der Antriebs- und Dosiervorrichtung heraus, verschieben. Eine erste Rücksetzfeder kann zwischen dem Betätigungsglied und dem Dosierglied angeordnet sein und z.B. sich daran abstützen. Alternativ oder zusätzlich kann eine zweite Rücksetzfeder zwischen einem Lagerelement und dem Produktbehälter oder dem Produktbehälterhalter angeordnet sein und sich z.B. daran abstützen.

Die Antriebs- und Dosiervorrichtung umfasst ferner ein Lagerelement, mit dem das Dosisanzeigeelement in einem Eingriff ist. Dieser Eingriff bewirkt vorteilhaft die Dreh- oder Schraubbewegung des Dosisanzeigeelements relativ zu der Zeigeeinrichtung. Z. B. kann der Eingriff zwischen Dosisanzeigeelement und Lagerelement ein Gewindeeingriff sein. Insbesondere kann das Lagerelement ein Außengewinde und das Dosisanzeigeelement ein Innengewinde aufweisen, wobei diese Gewinde ineinander greifen und dadurch bewirken, dass das Dosisanzeigeelement relativ zu dem Lagerelement schraubbar ist. Das Lagerelement kann z.B. drehfest mit dem Gehäuse verbunden sein oder in einem drehfesten Eingriff mit dem Gehäuse stehen, wobei bevorzugt ist, dass das Lagerelement relativ zu dem Gehäuse axial verschiebbar ist.

Insbesondere ist das Dosisanzeigeelement zwischen einer Maximaldosisposition und einer Nulldosisposition hin und her dreh- oder schraubbar. In der Nulldosisposition kann vorteilhaft die Dosis oder Ziffer "0" in der Zeigeeinrichtung ablesbar sein. In der Maximaldosisposition kann vorteilhaft die maximal mit der Antriebs- und Dosiervorrichtung ausschüttbare Produktdosis ablesbar sein.

In der Nulldosisposition kann das Dosisanzeigeelement gegen die Drehung in eine Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung bewegt werden, die eine Erhöhung der Dosis bewirkt. In der Maximaldosisposition ist das Dosisanzeigeelement vorzugsweise gegen die Drehung in eine Drehrichtung, nämlich in die Drehrichtung, die die Einstellung einer Dosis über die maximal einstellbare Dosis hinaus bewirken würde, blockiert. Das Dosisanzeigeelement kann in der Maximaldosisposition insbesondere nur in die Drehrichtung gedreht werden, die eine Verringerung der Produktdosis bewirkt.

Das Dosisanzeigeelement kann z. B. einen Anschlag aufweisen, der in der Nulldosisposition an einen Gegenanschlag anschlägt und somit die Drehung in eine Drehrichtung verhindert. Der gleiche oder ein zusätzlicher Anschlag des Dosisanzeigeelements kann die Drehung des Dosisanzeigeelements über die Maximaldosis hinaus verhindern. Insbesondere kann hierfür ein weiterer Gegenanschlag, nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung.

Das Lagerelement ist zusammen mit dem Dosisanzeigeelement relativ zu dem Gehäuse und entlang der Drehachse, insbesondere in distale Richtung verschiebbar. Alternativ kann das Dosisanzeigeelement ein Gewinde aufweisen, welches im Eingriff mit dem Gehäuse ist. Dadurch lässt sich das Dosisanzeigeelement zwar relativ zu dem Gehäuse hin und her schrauben, aber nicht unabhängig von der Schraubbewegung mit einer insbesondere reinen Axialbewegung verschieben.

Vorzugsweise ist das Betätigungsglied so mit dem Lagerelement gekoppelt, dass eine Verschiebung des Betätigungsglieds relativ zu dem Gehäuse und/oder dem Dosierglied eine Verschiebung des Lagerelements relativ zu dem Gehäuse und/oder dem Dosierglied insbesondere entlang der Längsachse der Antriebs- und Dosiervorrichtung bewirkt.

Dadurch dass das Dosisanzeigeelement in einem Eingriff mit dem Lagerelement ist und sich das Lagerelement relativ zu dem Gehäuse und entlang der Drehachse verschieben lässt, lässt sich auch das Dosisanzeigeelement relativ zu dem Gehäuse und entlang der Drehachse unabhängig von der Dreh- oder Schraubbewegung, welche das Dosisanzeigeelement bei der Dosiseinstellung ausführt, verschieben.

Vorteilhaft kann an der Zeigeeinrichtung und/oder an dem Dosisanzeigeelement abgelesen werden, dass das Lagerelement zusammen mit dem Dosisanzeigeelement verschoben wurde. Hierdurch lässt sich durch den Verwender kontrollieren, in welchem Betriebszustand sich die Antriebs- und Dosiervorrichtung befindet, d. h. ob die Antriebs- und Dosiervorrichtung insbesondere das Betätigungsglied für eine Ausschüttung betätigt oder unbetätigt ist.

In einer bevorzugten Variante kann das Betätigungsglied oder/und das Lagerelement zusammen mit dem Dosisanzeigeelement relativ zu der Zeigeeinrichtung, dem Gehäuse und entlang der Drehachse verschiebbar sein. Im Bereich der Zeigeeinrichtung, insbesondere in dem Fenster der Zeigeeinrichtung kann eine von der Dosisskala verschiedene Markierung erscheinen, wenn das Lagerelement verschoben ist. Die Markierung ist vorzugsweise an dem Dosisanzeigeelement angeordnet. Wenn das Lagerelement unverschoben ist, insbesondere die Antriebs- und Dosiervorrichtung oder das Betätigungsglied für die Produktausschüttung unbetätigt ist, kann die Markierung außerhalb der Zeigeeinrichtung angeordnet sein, wie z. B. von einem Gehäuse oder einem anderen Element verdeckt sein. Wird das Lagerelement verschoben, insbesondere die Antriebs- und Dosiervorrichtung für die Produktausschüttung betätigt, kann die Markierung aus dem abgedeckten Bereich hervortreten, so dass sie insbesondere an oder in der Zeigeeinrichtung erscheint oder ablesbar ist. Wird die Betätigung der Antriebs- und Dosiervorrichtung unterbrochen oder beendet, kann das Lagerelement in die Ursprungsposition zurückkehren, wodurch die Markierung vorzugsweise aus dem Bereich der Zeigeeinrichtung entfernt und insbesondere verdeckt wird.

Bei der Betätigung der Antriebs- und Dosiervorrichtung für eine Produktausschüttung kann die erste und/oder die zweite Kupplungs- oder Rückstellfeder gespannt werden. Mit anderen Worten ausgedrückt kann das Lagerelement bei der Betätigung gegen die Kraft dieser mindestens einen Feder verschoben werden, insbesondere aus einer unbetätigten Position in eine betätigte Position. Die Feder kann z. B. eine Schrauben- oder Wendelfeder sein, die als Druckfeder wirkt. Diese Feder bewirkt ferner, dass beim Unterbrechen oder Beenden der Betätigung das Lagerelement in seine Ausgangsposition oder unbetätigte Position zurückgesetzt wird. Insbesondere wird das Lagerelement bei der Betätigung in distale Richtung verschoben. Mittels der Feder wird das Lagerelement in proximale Richtung zurück verschoben, wenn die Betätigung unterbrochen oder beendet wird.

Die Betätigung des Betätigungsglieds bewirkt insbesondere, dass das Lagerelement zusammen mit dem Dosisanzeigeelement relativ zu dem Gehäuse und entlang der Drehachse verschoben wird. Im weiteren Sinne kann die Betätigung des Betätigungsglieds bewirken, dass ein Vortriebsglied, dessen distales Ende vorgesehen ist, auf einen Kolben des an der Antriebs- und Dosiervorrichtung befestigten oder befestigbaren Produktbehälters zu wirken, in distale Richtung, insbesondere Ausschüttrichtung verschoben wird. Das Betätigungsglied kann z. B. an dem proximalen, d. h. hinteren Ende der Antriebs- und Dosiervorrichtung angeordnet sein oder das proximale Ende der Antriebs- und Dosiervorrichtung bilden. Alternativ kann das Betätigungselement seitlich am Gehäuse und/oder zwischen dem distalen Ende und dem proximalen Ende der Antriebs- und Dosiervorrichtung angeordnet sein. Allgemein kann das Betätigungsglied in der Art eines Betätigungsknopfs ausgebildet sein. Das Betätigungsglied wird bei der Betätigung vorzugsweise relativ zu dem Gehäuse oder dem Dosierglied verschoben. Insbesondere kann der Verwender der Vorrichtung das Betätigungsglied vorteilhaft z.B. mit dem Daumen seiner Hand, welche das Gehäuse der Antriebs- und Dosiervorrichtung umgreift, betätigen.

Das Betätigungsglied ist vorzugsweise mit dem Lagerelement so verbunden, dass es das Lagerelement bei Betätigung verschiebt, insbesondere über ein Kupplungsglied, das z. B. axialfest und drehbar mit dem Lagerelement verbunden sein kann. Das Kupplungsglied kann alternativ zu einer axialfesten Verbindung an das Lagerelement lose anstoßen (und umgekehrt), so dass das Kupplungsglied das Lagerelement mitnehmen kann, und umgekehrt.

In bevorzugten Ausführungen weist das Lagerelement wenigstens einen federnd angeordneten Schnapper auf, an dem ein Kupplungsglied an einer Anstoßstelle, insbesondere stirnseitig, anstößt, wenn das Betätigungsglied für eine Produktausschüttung betätigt ist. Der Schnapper ist mittels des Dosisanzeigeelements aus der Position elastisch auslenkbar, in der er vor dem Lagerelement angeordnet ist und das Lagerelement an den Schnapper anstoßen kann. Durch dessen Auslenkung, bevorzugt zur Längsachse der Vorrichtung hin, ist das Lagerelement zu dem Kupplungsglied hin verschiebbar oder/ und der Schnapper an der Anstoßstelle insbesondere axial vorbeischiebbar, insbesondere wenn das Betätigungsglied betätigt ist. Die Verschiebung kann z.B. von der oder einer der Rücksetzfedern, wie z.B. der zweiten Rücksetzfeder, bewirkt werden, die auf das Lagerelement wirkt. Vorteilhaft schlägt das Lagerelement bei dieser Bewegung an einem Anschlag an, so dass ein taktiles und/oder akustisches Signal, wie z.B. ein einzelnes oder einziges Klickgeräusch, erzeugt wird, das dem Verwender z.B. das Ende der Produktausschüttung signalisiert.

Vorzugsweise weist das Dosisanzeigeelement z.B. an seinem Innenumfang eine Abragung, insbesondere einen Nocken auf, die so angeordnet ist, dass sie den Schnapper auslenkt, kurz bevor das Dosisanzeigeelement seine Nulldosisposition erreicht, insbesondere an den Nulldosisgegenanschlag anschlägt. Dadurch wird die Signalerzeugung ausgelöst. Der Nocken kann den Schnapper auslenken, z.B. wenn die in der Zeigeeinrichtung ablesbare Dosis kleiner als 10 IU vorzugsweise kleiner als 5 oder kleiner gleich 2 IU ist Bevorzugt wird der Schnapper ausgelenkt, wenn 1 oder 2 IU durch die Zeigeeinrichtung laufen, insbesondere herunterzählen.

In allgemein bevorzugten Ausführungen kann die Betätigung des Betätigungsglieds bewirken, dass das Dosisanzeigeelement relativ zu oder an dem Lagerelement oder dem Gehäuse gedreht, insbesondere geschraubt wird, insbesondere in eine Richtung, dass die sich bei der Drehbewegung an der Zeigeeinrichtung vorbei bewegenden Werte der Dosisskala zurückzählen. Vorzugsweise stehen der Drehwinkel des Dosisanzeigeelements und der Ausschütthub des Vortriebsglieds in einem proportionalen Zusammenhang, insbesondere zu jedem Zeitpunkt während der Dosisausschüttung. Hierdurch lässt sich eine Echtzeitanzeige verwirklichen, die bei der Dosisausschüttung zurückzählt, bis sie schließlich beim Wert 0 angelangt, wobei die Ausschüttung dieser Dosis dann beendet ist. Wird die Betätigung für die Ausschüttung während des Zurückdrehens des Dosisanzeigeelements unterbrochen, zeigt das Dosisanzeigeelement die noch für die Ausschüttung dieser Dosis erforderliche Restmenge an.

In einer bevorzugten alternativen Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie automatisch, insbesondere durch eine in der Antriebs- und Dosiervorrichtung enthaltene Feder, insbesondere Ausschüttfeder, in der die benötigte Energie gespeichert ist oder werden kann, bereitgestellt wird. Z. B. kann die in der Ausschüttfeder gespeicherte Federenergie bei Betätigung des Betätigungsglieds an das Dosisanzeigeelement oder/und das Vortriebsglied abgegeben werden, so dass das Dosisanzeigeelement zurückgedreht und das Vortriebsglied in distale Richtung verschoben werden. Die Ausschüttfeder ist vorzugsweise so mit dem Dosierglied, insbesondere über eine lösbare Kupplung, gekoppelt, dass eine Drehung des Dosierglieds bei der Dosiseinstellung, insbesondere bei einer Dosiserhöhung oder Drehung in die erste Drehrichtung, die Ausschüttfeder vorspannt. Die Ausschüttfeder kann dann die für die eingestellte Dosis erforderliche Energie speichern.

Bei Drehung des Dosierglieds in eine zweite Drehrichtung, die eine Dosisverringerung beiwirkt, kann entweder bewirken, dass die Ausschüttfeder entspannt oder nicht entspannt wird. Soll die Ausschüttfeder bei Drehung des Dosierglieds in die zweite Drehrichtung entspannt werden, kann ein Ende der Feder in beide Drehrichtungen drehfest, insbesondere über die Kupplung, mit dem Dosierglied gekoppelt sein. Soll die Ausschüttfeder bei Drehung des Dosierglieds in die zweite Drehrichtung nicht entspannt werden, ist es vorteilhaft, insbesondere kinematisch zwischen dem Dosierglied und der Feder, insbesondere zusätzlich zu der genannten Kupplung, eine unidirektionale Kupplung, insbesondere eine Ratsche anzuordnen, welche die Drehung des Dosierglieds in die erste Drehrichtung an die Ausschüttfeder übertragt und die Drehung des Dosierglieds in die zweite Drehrichtung an die Ausschüttfeder nicht überträgt.

Um zu verhindern, dass die durch Drehung des Dosierglieds vorgespannte Ausschüttfeder ihre in ihr gespeicherte Energie an das Dosierglied abgibt, wodurch das Dosierglied beim Loslassen automatisch in die zweite Drehrichtung zurückgedreht werden würde, ist vorzugsweise zwischen dem Dosierglied und dem Gehäuse eine Ratsche, insbesondere eine Ratschenfeder angeordnet, die z.B. nach dem Prinzip einer Rutschkupplung wirkt. Die Ratsche ist so ausgelegt, dass sie ein die Drehung des Dosierglieds hemmendes Moment erzeugt, das größer ist als das von der, z.B. in der Maximaldosisposition des Dosisanzeigeelements maximal vorgespannten Ausschüttfeder auf das Dosierglied ausgeübte Drehmoment. Nur durch zusätzliches Aufbringen eines Drehmoments durch den Verwender lässt sich das Dosierglied drehen.

Die Ratsche kann eine Ratschenfeder umfassen, die z.B. drehfest mit dem Dosierglied verbunden oder an dem Dosierglied befestigt ist und in eine Verzahnung, insbesondere eine Stirnverzahnung, des Gehäuses eingreift. Die Verzahnung weist mehrere über den Umfang angeordnete Zähne auf, die vorteilhaft in solch einem Raster voneinander beabstandet sind, dass jedes Raster 1 IU oder 2 IU entspricht. Die Ratschenfeder kann Eingriffsnocken gegen die Verzahnung drücken, wobei der Eingriffsnocken bei Drehung des Dosierglieds über die Verzahnung rastet, insbesondere in den 1 IU- bzw. 2 IU-Schritten. Die Ratschenfeder kann den Eingriffsnocken z.B. bilden und/oder ringförmig und/oder ein Stanz-Biege-Teil sein, welches aus einem z.B. plattenförmigen Halbzeug ausgestanzt wurde.

Das Dosierglied, insbesondere der Dosierknopf kann das Betätigungsglied, insbesondere den Betätigungsknopf umgeben oder aufnehmen. Somit können das Dosierglied und das Betätigungsglied das proximale Ende der Antriebs- und Dosiervorrichtung bilden. Vorzugsweise ist das Betätigungsglied für die Betätigung relativ zu dem Dosierglied verschiebbar.

Die Antriebs- und Dosiervorrichtung kann ein Vortriebsglied aufweisen, dessen distales Ende vorgesehen ist, auf einen Kolben, insbesondere mittelbar oder vorzugsweise unmittelbar zu wirken. Der Kolben kann Teil eines an der Antriebs- und Dosiervorrichtung befestigten oder befestigbaren Produktbehälters, wie z. B. einer Karpule sein. Das Vortriebsglied kann im weiteren Sinne als Kolbenstange bezeichnet oder ausgestaltet werden, wobei das Vortriebsglied nicht notwendigerweise massiv sein muss, sondern auch hohl, wie z. B. hülsenförmig ausgestaltet sein kann. An dem distalen Ende des Vortriebsglieds kann optional ein z. B. drehbarer Flansch gebildet sein, der gegen den Kolben drückt. Allgemein ist es bevorzugt, dass das distale Ende des Vortriebsglieds gegen den Kolben drückt. Das Vortriebsglied ist vorzugsweise relativ zu dem Gehäuse entlang der Längsachse der Antriebs- und Dosiervorrichtung verschiebbar.

Das Vortriebsglied weist vorzugsweise ein Außengewinde und eine dem Außengewinde überlagerte Längsführung auf, wobei die Vorrichtung ein Rotationsglied aufweist, welches in eines aus Längsführung und Gewinde eingreift, und wobei das andere aus Längsführung und Gewinde in einem drehfesten Eingriff mit dem Gehäuse oder einem gehäusefesten Element ist. Das Rotationsglied ist vorzugsweise axialfest in dem Gehäuse angeordnet. Greift das Rotationsglied in die Längsführung ein, wird das Vortriebsglied mit dem Rotationsglied mitgedreht, wodurch sich das Rotationsglied aufgrund des Gewindeeingriffs entlang seiner Längsachse schraubt. Greift das Rotationsglied in das Gewinde ein, wird das Vortriebsglied relativ zu dem Rotationsglied geschraubt, wodurch das Rotationsglied aufgrund des drehfesten Eingriffs mit dem Gehäuse oder dem gehäusefesten Element entlang seiner Längsachse geschoben wird.

Das in das Vortriebsglied eingreifende Element, insbesondere eine Innenhülse, kann vorzugsweise das Vortriebsglied umgeben, wie z. B. hülsenförmig umgeben und/oder gehäusefest sein oder von dem Gehäuse gebildet werden. Zwischen diesem hülsenförmigen Gehäuseteil und einem äußeren, vorzugsweise ebenfalls hülsenförmigen Gehäuseteil kann ein Ringspalt gebildet sein, der den Vorteil birgt, dass ein optional vorhandenes Dosisanzeigeelement, insbesondere Dosisanzeigetrommel, darin aufgenommen sein kann.

Dies bewirkt, dass die Länge der Antriebs- und Dosiervorrichtung gering gehalten werden kann.

Die Drehung des Rotationsglieds bewirkt insbesondere, dass die Feder Energie an das Vortriebsglied abgibt, wodurch das Vortriebsglied in distale Richtung bewegt wird. Die Drehung des Rotationsglieds um einen bestimmten Drehwinkel bewirkt den Vorschub des Vortriebsglieds um einen bestimmten Ausschütthub. Durch selektive Freigabe oder Sperrung einer Drehung des Rotationsglieds relativ zu dem Gehäuse kann der Ausschüttfeder erlaubt werden, dass sie das Vortriebsglied relativ zu Gehäuse in distale Richtung bewegen kann oder nicht bewegen kann. Insbesondere kann das Rotationsglied so mit dem Betätigungsglied gekoppelt sein, dass es bei der Betätigung des Betätigungsglieds für eine Produktausschüttung für eine Drehung relativ zu dem Gehäuse freigegeben ist und bei Nichtbetätigung des Betätigungsglieds für eine Rotation relativ zu dem Gehäuse gesperrt ist. Insbesondere kann zwischen Betätigungsglied und Rotationsglied eine Kupplung angeordnet sein, welche die Freigabe und Sperrung der Drehung des Rotationsglieds relativ zu dem Gehäuse bewirkt.

Vorteilhaft kann die Kupplung durch Betätigen des Betätigungsglieds die Drehung des Rotationsglieds relativ zu dem Gehäuse freigeben und durch Loslassen des Betätigungsglieds die Drehung des Rotationsglieds relativ zu dem Gehäuse blockieren.

Es ist vorteilhaft, dass das Rotationsglied mittels einer Ratsche mit dem Gehäuse verbunden ist. Das Rotationsglied kann an seinem Umfang und/oder seinem distalen Ende ein federndes Rastglied aufweisen, welches in eine sich über den Umfang erstreckende Verzahnung, insbesondere Innenverzahnung, die z.B. von dem Gehäuse oder einem gehäusefesten Element, wie z.B. einer Innenhülse des Gehäuses gebildet wird, eingreift. Dies bewirkt einerseits, dass ein gewisses Drehmoment auf das Rotationsglied ausgeübt werden muss, um das Rotationsglied relativ zu dem Gehäuse zu drehen und anderseits, dass die Drehung des Rotationsglieds durch Klickgeräusche signalisiert wird. Somit kann die Produktausschüttung mit dem sich drehenden Rotationsglied dem Verwender signalisiert werden.

Das Dosisanzeigeelement, insbesondere die Dosisanzeigetrommel kann während des Einstellens einer Dosis, d. h. im unbetätigten Zustand der Antriebs- und Dosiervorrichtung oder des Betätigungsglieds relativ zu dem Rotationsglied drehbar sein. Vorzugsweise ist das Dosisanzeigeelement während der Betätigung der Vorrichtung zum Ausschütten der Produktdosis relativ zu dem Rotationsglied drehfest und z.B. axial bewegbar, oder drehfest mit dem Rotationsglied verbunden, insbesondere mit der beschriebenen Kupplung oder einer weiteren Kupplung.

Die Antriebs- und Dosiervorrichtung kann eine z.B. erste Kupplung, insbesondere eine Ausschüttkupplung, aufweisen, welche bei unbetätigtem Betätigungsglied geöffnet und bei betätigtem Betätigungsglied geschlossen ist oder durch Betätigung des Betätigungsglieds geschlossen wird. Bei geöffneter Kupplung ist das Kupplungsglied relativ zu dem Rotationsglied drehbar, insbesondere bei der Dosiseinstellung. Bei geschlossener Kupplung ist das Rotationsglied relativ zu dem Kupplungsglied drehfest, so dass das Rotationsglied eine Drehbewegung des Kupplungsglieds mitmacht. Zur Bildung dieser Kupplung können das Kupplungsglied eine erste Kupplungsstruktur und das Rotationsglied eine zweite Kupplungsstruktur aufweisen, die bei geöffneter Kupplung außer Eingriff und bei geschlossener Kupplung im Eingriff mit der ersten Kupplungsstruktur ist. Die Kupplung kann eine Klauenkupplung sein. Die erste Kupplungsstruktur und die zweite Kupplungsstruktur können jeweils eine Verzahnung aufweisen, wobei die Verzahnungen zur Bildung einer drehfesten Verbindung ineinander eingreifen können. Beispielsweise ist die erste Kupplungsstruktur eine Außenverzahnung am proximalen Ende des Rotationsglieds. Beispielsweise ist die zweite Kupplungsstruktur eine Innenverzahnung am hülsenförmigen Kupplungsglied.

Die Antriebs- und Dosiervorrichtung kann eine z.B. zweite Kupplung, insbesondere eine Dosierkupplung, aufweisen, welche bei unbetätigtem Betätigungsglied geschlossen und bei betätigtem Betätigungsglied geöffnet ist oder durch Betätigung des Betätigungsglieds geöffnet wird. Bei geöffneter Kupplung ist das Kupplungsglied relativ zu dem Dosierglied drehbar, insbesondere bei der Produktausschüttung. Bei geschlossener Kupplung ist das Dosierglied relativ zu dem Kupplungsglied drehfest, so dass das Kupplungsglied eine Drehbewegung des Rotationsglieds mitmacht, wodurch die Ausschüttfeder gespannt wird. Zur Bildung dieser Kupplung können das Kupplungsglied eine dritte Kupplungsstruktur und das Dosierglied eine vierte Kupplungsstruktur aufweisen, die bei geöffneter Kupplung außer Eingriff und bei geschlossener Kupplung im Eingriff mit der dritten Kupplungsstruktur ist. Die Kupplung kann eine Klauenkupplung sein. Die dritte Kupplungsstruktur und die vierte Kupplungsstruktur können jeweils eine Verzahnung aufweisen, wobei die Verzahnungen zur Bildung einer drehfesten Verbindung ineinander eingreifen können. Beispielsweise ist die dritte Kupplungsstruktur eine Außenverzahnung am Umfang des Kupplungsglieds. Beispielsweise ist die vierte Kupplungsstruktur eine Innenverzahnung am hülsenförmigen Dosierglied.

Besonders vorteilhaft ist es, wenn das Kupplungsglied mittels der ersten Kupplung bereits drehfest mit dem Rotationsglied gekoppelt ist und mittels der zweiten Kupplung noch drehfest mit dem Dosierglied verbunden ist, während das Betätigungsglied für die Betätigung in Bezug auf das Gehäuse verschoben wird. Hierdurch wird sichergestellt, dass das Kupplungsglied sicher mit dem Rotationsglied gekoppelt ist, wenn das Kupplungsglied für eine Drehung relativ zu dem Gehäuse oder dem Dosierglied freigegeben ist. Mit anderen Worten gibt es zwischen der vollständig betätigten Position und der unbetätigten Position des Betätigungsglieds eine Zwischenposition, in der das Kupplungsglied sowohl drehfest mit dem Dosierglied als auch drehfest mit dem Rotationsglied gekoppelt ist.

Die Antriebs- und Dosiervorrichtung kann eine z.B. dritte Kupplung aufweisen, welche bei unbetätigtem Betätigungsglied geschlossen und bei betätigtem Betätigungsglied geöffnet ist oder durch Betätigung des Betätigungsglieds geöffnet wird. Diese Kupplung koppelt das Rotationsglied verdrehfest mit dem Gehäuse, wenn sie geschlossen, d.h. das Betätigungsglied unbetätigt ist, wobei die Kupplung mittels Betätigung des Betätigungsglieds lösbar ist bzw. geöffnet wird, so dass das Rotationsglied relativ zu dem Gehäuse drehbar ist.

Zur Bildung dieser Kupplung können das Rotationsglied eine fünfte Kupplungsstruktur und das Dosierglied eine sechste Kupplungsstruktur aufweisen, die bei geöffneter Kupplung außer Eingriff und bei geschlossener Kupplung im Eingriff mit der fünften Kupplungsstruktur ist. Die Kupplung kann eine Klauenkupplung sein. Die fünfte Kupplungsstruktur und die sechste Kupplungsstruktur können jeweils eine Verzahnung aufweisen, wobei die Verzahnungen zur Bildung einer drehfesten Verbindung ineinander eingreifen können. Beispielsweise ist die fünfte Kupplungsstruktur eine Außenverzahnung am Umfang des Rotationsglieds.

Beispielsweise ist die sechste Kupplungsstruktur eine Innenverzahnung am Gehäuse oder einem gehäusefesten Element.

Besonders vorteilhaft ist es, wenn - während das Betätigungsglied für die Betätigung in Bezug auf das Gehäuse verschoben wird - die dritte Kupplung erst dann geöffnet wird, wenn die erste Kupplung geschlossen und die zweite Kupplung geöffnet ist.

Optional kann die Antriebs- und Dosiervorrichtung ein Ratschenelement, insbesondere eine Ratschenhülse aufweisen. Das Ratschenelement ist vorzugsweise mit einem Ende der Antriebsfeder drehfest verbunden, insbesondere ist das Ende der Feder an dem Ratschenelement befestigt. Somit kann sich die Ausschüttfeder mit einem, insbesondere ihrem proximalen Ende drehfest an dem Ratschenelement abstützen. Das Ratschenelement kann ein erstes federnd angeordnetes Rastelement und ein zweites federnd angeordnetes Rastelement aufweisen, insbesondere bilden. Die Ratschenhülse ist vorzugsweise zwischen dem Rotationsglied und dem Kupplungsglied angeordnet. Das erste Rastelement kann in solch einem ersten Rasteingriff mit dem Kupplungsglied, insbesondere einer Innenverzahnung des Kupplungsglieds, sein, der eine Drehbewegung des Kupplungsglieds in eine Drehrichtung, insbesondere in die Drehrichtung, welche ein Spannen der Feder bewirkt, auf das Ratschenelement überträgt und in die entgegengesetzte Drehrichtung, welche ein Entspannen der Feder bewirken würde, nicht überträgt. Hierdurch wird erreicht, dass eine Drehung des Dosierglieds die Antriebsfeder nicht entspannen kann. Das zweite Rastelement kann in solch einem zweiten Rasteingriff mit dem Rotationsglied, insbesondere einer Außenverzahnung des Rotationsglieds, sein, der eine Drehbewegung des Ratschenelements realtiv zu dem Rotationsglied in eine Drehrichtung, insbesondere in die Drehrichtung, welche ein Spannen der Feder bewirkt, zulässt und in die entgegengesetzte Drehrichtung, insbesondere die Drehrichtung, in welche sich das Rotationsglied für eine Produktausschüttung dreht, sperrt. Hierdurch wird erreicht, dass die Feder ihr Drehmoment über das Ratschenelement und den zweiten Rasteingriff für die Produktausschüttung an das Rotationsglied abgibt.

Vorzugsweise ist der erste Rasteingriff so ausgelegt, dass das Kupplungsglied bei Drehung in die Drehrichtung, welche ein Spannen der Feder bewirken würde, relativ zu dem Ratschenelement drehbar ist, wenn die Antriebsfeder so stark vorgespannt, ist, dass ein Grenzmoment erreicht ist oder die Antriebsfeder vollständig vorgespannt ist. Hierdurch wird die Vorrichtung einerseits vor Beschädigung durch eine zu stark vorgespannte Feder geschützt und andererseits sichergestellt, dass eine Dosierung, d.h. Drehung des Dosierglieds noch möglich ist, auch wenn die Antriebsfeder vollständig vorgespannt ist.

In allgemein bevorzugten Ausführungen kann das Dosisanzeigeelement einen Anschlag, wie z. B. einen Nulldosisanschlag aufweisen, der von einem Gegenanschlag, insbesondere einem Nulldosisgegenanschlag wegbewegt wird, wenn eine Dosiserhöhung vorgenommen wird, und der zu dem Gegenanschlag hinbewegt wird, wenn eine Dosisverringerung vorgenommen wird, oder wenn die Vorrichtung für die Ausschüttung der eingestellten Produktdosis betätigt wird.

In vorteilhaften Weiterbildungen kann die Antriebs- und Dosiervorrichtung einen Mechanismus zum Verhindern des Einstellens einer Dosis, welche die Menge eines Medikaments in dem Produktbehälter übersteigt, aufweisen. Insbesondere kann dieser Mechanismus die Drehung des Dosierglieds in eine Richtung, welche eine Dosiserhöhung bewirken würde blockieren, insbesondere auch wenn der Maximaldosisanschlag des Dosisanzeigeelements und der Maximaldosisgegenanschlag noch nicht in einem Eingriff sind oder wenn in der Zeigeeinrichtung eine Dosis angezeigt wird, die kleiner ist als die maximal einstellbare Produktdosis. Der Mechanismus verhindert somit, dass eine Dosis eingestellt werden kann, welche die Restmenge des in dem Produktbehälter enthaltenen Produkts übersteigt, wodurch die Gefahr einer Fehlanwendung der Antriebs- und Dosiervorrichtung verringert wird. Der Mechanismus kann z. B. einen Begrenzer aufweisen, der zwischen zwei Teilen angebracht ist, von denen sich eines relativ zu dem anderen während der Dosiseinstellung dreht und bei der Betätigung, d. h. der Dosisausschüttung nicht dreht. Z. B. kann der Begrenzer zwischen dem Dosiseinstellglied, das insbesondere als Dosiseinstellknopf oder Dosiseinstellhülse ausgestaltet sein kann, und dem Gehäuse oder einem gehäusefesten Element angeordnet sein. Der Begrenzer, das Dosiseinstellglied und das Gehäuse können so miteinander gekoppelt sein, dass eine relative Drehung, insbesondere während der Dosiseinstellung, zwischen dem Dosiseinstellglied und dem Gehäuse bewirkt, dass sich der Begrenzer zu einer Stoppposition hinbewegt, in welcher der Begrenzer das Einstellen einer Dosis verhindert, die die Menge eines Produkts in dem Produktbehälter übersteigt. Beispiele für entsprechend geeignete Begrenzer werden in der WO 2010/149209 A1 oder in der WO 01/19434 A1, insbesondere in deren Figur 3 offenbart. Beispielsweise kann der Begrenzer ein Innengewinde aufweisen, welches in dem Eingriff mit einem Außengewinde des Gehäuses ist. Insbesondere kann der Begrenzer an seiner Außenseite eine Längsführung aufweisen, mit der er in einem Eingriff mit dem Dosiseinstellglied ist, so dass das Dosiseinstellglied relativ zu dem Begrenzer drehfest ist. Alternativ kann das Gehäuse die Längsführung für den Begrenzer aufweisen, so dass der Begrenzer relativ zu dem Gehäuse drehfest ist und kann der Begrenzer ein Gewinde, insbesondere Außengewinde aufweisen, welches in einen Gewinde, insbesondere Innengewinde des Dosiseinstellglieds eingreift.

Die Stoppposition wird durch einen Anschlag für den Begrenzer definiert, wobei der Anschlag von dem Gehäuse oder dem Dosiseinstellglied oder einem zumindest axial oder in Umfangsrichtung gehäusefesten Mittel gebildet werden kann. Sind der Begrenzer und der Anschlag in einem Kontakt, ist eine Drehung des Dosiseinstellglieds in eine Drehrichtung, welche eine Erhöhung der Dosis bewirken würde, nicht mehr möglich oder blockiert.

In allgemein bevorzugten Weiterbildungen kann die Antriebs- und Dosiervorrichtung mindestens einen Signalerzeugungsmechanismus, wie z.B. die zwischen dem Rotationsglied und dem Gehäuse oder dem gehäusefesten Element gebildete Ratsche, aufweisen, der angepasst ist, während der Dosiseinstellung oder/und der Produktausschüttung ein akustisches und/oder taktiles Signal, insbesondere mechanisch, zu erzeugen. Ein solches Signal kann insbesondere als Klicksignal wahrgenommen werden. Zum Beispiel kann ein (erster) Signalerzeugungsmechanismus vorgesehen sein, der das Signal während der Dosiseinstellung erzeugt und optional als Dosiseinstellungssignalerzeugungsmechanismus bezeichnet werden kann. Ferner kann ein weiterer (zweiter) Signalerzeugungsmechanismus vorgesehen sein, der das Signal während der Produktausschüttung erzeugt und optional als Produktausschüttungssignalerzeugungsmechanismus bezeichnet werden kann. Alternativ kann ein (gemeinsamer) Signalerzeugungsmechanismus vorgesehen sein, der das Signal während der Dosiseinstellung und während der Produktausschüttung erzeugt.

Der Signalerzeugungsmechanismus kann allgemein zwischen zwei Teilen angeordnet sein, die sich bei der Dosiseinstellung oder/und der Produktausschüttung relativ zueinander bewegen, insbesondere drehen. Eines der Teile kann ein z.B. federnd angeordnetes Rastglied aufweisen, das in eine z.B. über den Umfang angeordnete Verzahnung des anderen der zwei Teile eingreift. Wird ein Teil relativ zu dem anderen verdreht, kann das Rastglied über die Verzahnung gleiten und dabei das Signal erzeugen. Die Verzahnung kann von einem Innenumfang oder einem Außenumfang oder einer Stirnfläche des Teils gebildet sein.

Die Erfindung wurde anhand mehrerer vorteilhafter Ausführungsformen beschrieben. Im Folgenden werden vorteilhafte Ausführungsformen anhand von Figuren beschrieben. Die dabei offenbarten Merkmale bilden den Gegenstand der Ansprüche, insbesondere der unabhängigen Ansprüche, insbesondere einzeln und in jeglicher Kombination mit den Ansprüchen oder/und der vorhergehenden Beschreibung vorteilhaft weiter. Es zeigen:
- Figur 1: eine Explosionsdarstellung der Einzelteile einer ersten Ausführungsform einer erfindungsgemäßen Antriebs- und Dosiervorrichtung,
- Figuren 2a-d: die Antriebs- und Dosiervorrichtung aus Figur 1 in einem Ausgangs- oder Auslieferungszustand, wobei Figur 2b eine Schnittansicht der Figur 2a ist und Figur 2c eine um 90° und Figur 2d eine um 45° gedrehte Schnittansicht der Figur 2a entlang der Längsachse der Vorrichtung ist,
- Figuren 3a-d: die Injektionsvorrichtung in den Ansichten aus den Figur 2a-d in einem Zustand, in dem die maximal einstellbare Dosis eingestellt ist,
- Figuren 4a-d: die Ansichten aus Figuren 2a-d, jedoch in einem Zustand, bei dem die in den Figuren 3a-d eingestellte Dosis vollständig ausgeschüttet wurde und ein Betätigungsglied noch betätigt ist,
- Figur 5: eine Explosionsdarstellung der Einzelteile einer zweiten Ausführungsform einer erfindungsgemäßen Antriebs- und Dosiervorrichtung,
- Figuren 6a-c: die Antriebs- und Dosiervorrichtung aus Figur 5 in einem Ausgangs- oder Auslieferungszustand, wobei Figur 6b eine Schnittansicht der Figur 6a ist und Figur 6c eine um 90° gedrehte Schnittansicht der Figur 6a entlang der Längsachse der Vorrichtung ist,
- Figuren 7 und 8: Explosionsdarstellungen der Einzelteile einer dritten Ausführungsform einer erfindungsgemäßen Antriebs- und Dosiervorrichtung, wobei Figur 8 eine Schnittdarstellung der Figur 7 ist, und
- Figuren 9a-c: die Antriebs- und Dosiervorrichtung aus den Figuren 7 und 8 in einem Ausgangs- oder Auslieferungszustand, wobei Figur 9b eine Schnittansicht der Figur 9a ist und Figur 9c eine um 90° gedrehte Schnittansicht der Figur 9a entlang der Längsachse der Vorrichtung ist.

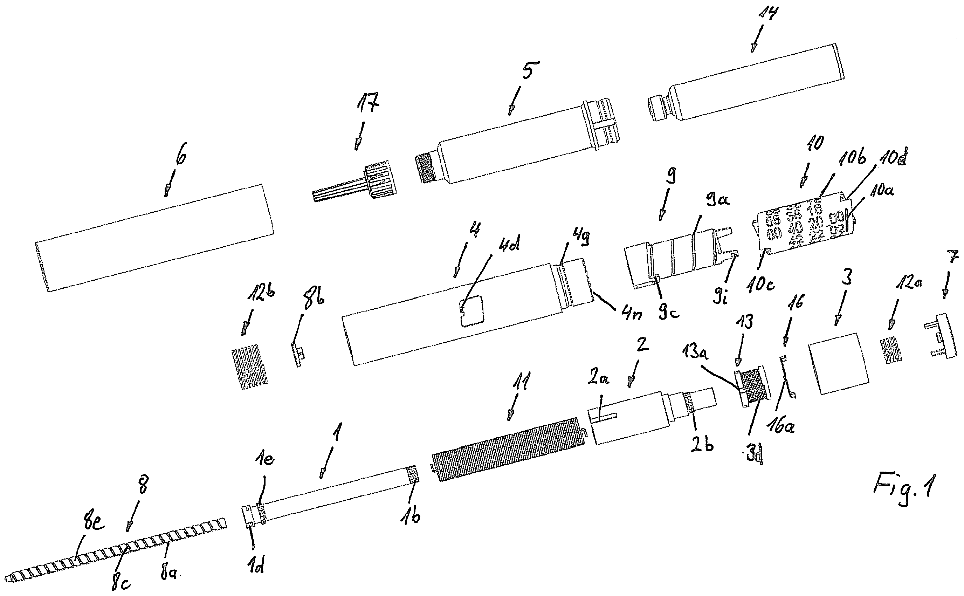

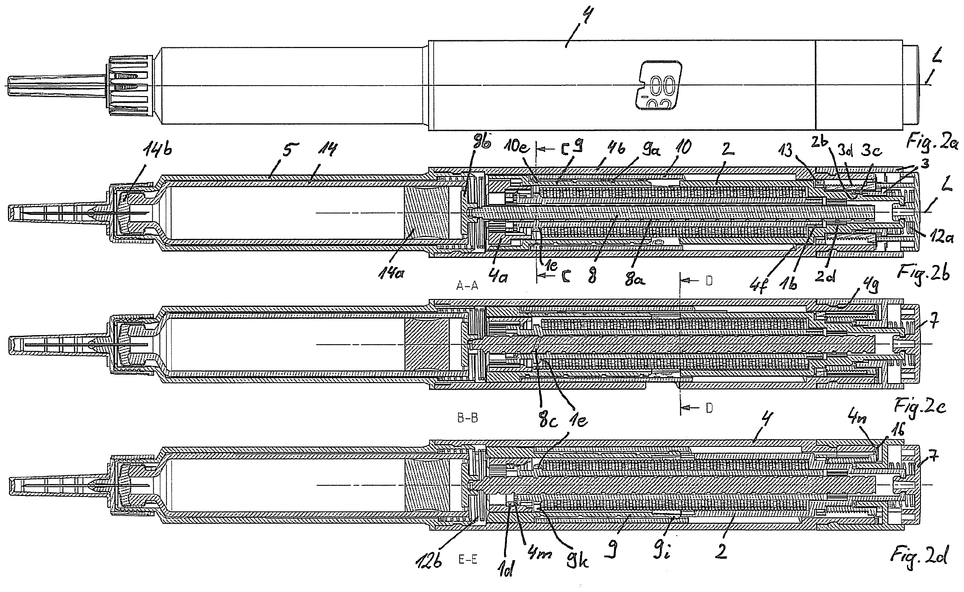

Die Antriebs- und Dosiervorrichtung umfasst in einer ersten Ausführungsform, die in den Figuren aus den Figuren 1 bis 4d dargestellt wird, ein hülsenförmiges Gehäuse 4 auf, welches eine Außenhülse 4b aufweist, welche vom Verwender mit einer Hand umgreifbar ist. Wie z.B. aus Figur 2b erkennbar ist, umfasst das Gehäuse 4 ferner eine Innenhülse 4a, die ein Widerlager für eine Ausschüttfeder 11 bildet und konzentrisch zu der Außenhülse 4b angeordnet ist. Innenhülse 4a und Außenhülse 4b sind über mehrere über den Umfang verteilte, voneinander beabstandete Stege miteinander verbunden. Zwischen der Außenhülse 4b und der Innenhülse 4a ist ein von den Stegen unterbrochener Ringspalt gebildet, durch den distale Abschnitte eines Lagerelements 9 ragen. Zwischen dem Lagerelement 9 und der Außenhülse 4b ist ein weiterer Ringspalt gebildet, in dem ein Dosisanzeigeelement 10, das insbesondere als Dosisanzeigetrommel, d. h. hülsenförmig ausgestaltet ist, angeordnet ist.

An dem distalen Ende des Gehäuses 4 ist eine hülsenförmige Produktbehälteraufnahme 5 aus einem vorzugsweise transparenten Material angeordnet, in der ein Produktbehälter 14 in der Gestalt einer Karpule aufgenommen ist. Der Produktbehälter 14 ist mittels der Produktbehälteraufnahme 5 mit dem Gehäuse 4 unlösbar verbunden, so dass die Antriebs- und Dosiervorrichtung insbesondere zusammen mit der Produktbehälteraufnahme 5 und dem Produktbehälter 14 eine Einweg-Injektionsvorrichtung bildet, die nach vollständiger Entleerung des Produktbehälters 14 als ganzes entsorgt wird. Der Produktbehälter 14 weist an seinem distalen Ende ein Septum 14b auf, welches von einer durch am distalen Ende des Produktbehälters 14 bzw. der Produktbehälteraufnahme 5 anbringbaren Nadel durchstechbar ist. In dem Produktbehälter 14 ist ein Kolben 14a aufgenommen, wobei das auszuschüttende Produkt zwischen dem Septum 14b und dem Kolben 14a angeordnet ist. Eine Verschiebung des Kolbens 14a in Richtung Septum oder in distale Richtung, mithin Ausschüttrichtung, bewirkt eine Ausschüttung des in dem Produktbehälter 14 enthaltenen Produkts. In Figur 1 wird zusätzlich noch eine Schutzkappe 6 dargestellt, welche über der Produktbehälteraufnahme 5 anbringbar ist und vor dem Injizieren einer Dosis abgenommen wird. Die Nadel kann Teil einer Nadeleinheit 17 sein, von der Teile, wie z.B. eine Nadelabdeckkappe, nach dem Anbringen der Nadel und von der Nadel entfernt werden.

Das Gehäuse 4, insbesondere die Innenhülse 4a ist in einem Eingriff mit einem Vortriebsglied 8, welches auch als Stößel oder Kolbenstange bezeichnet werden kann. Der Eingriff ist dergestalt, dass das Vortriebsglied 8 relativ zu dem Gehäuse 4 drehfest und axial entlang der Längsachse L verschiebbar ist. Das Vortriebsglied 8 weist ein Außengewinde 8c und eine das Außengewinde 8c überlagerte Längsführung 8a, wie z.B. eine Längsnut oder einen abgeflachten Bereich, auf. Das Gehäuse 4 ist in dem verdrehfesten Eingriff mit der Längsführung 8a. In das Außengewinde des Vortriebsglieds 8 greift ein Innengewinde eines hülsenförmigen Rotationsglieds 1 ein, wobei das Rotationsglied 1 drehbar und axialfest in dem Gehäuse 4 angeordnet oder gelagert ist. Eine Drehung des Rotationsglieds 1 bewirkt, dass das Vortriebsglied 8 entlang der Längsachse L verschoben wird, insbesondere in Richtung Kolben 14a. Das Vortriebsglied 8 ist so angeordnet, dass sein distales Ende, das durch einen aufgesteckten Flansch 8b gebildet wird, auf den Kolben 14a wirken, insbesondere gegen den Kolben 14a drücken kann.

Die Innenhülse 4a weist über ihren Umfang eine Innenverzahnung 4m auf, in die ein Rastglied 1d eingreift, das federnd am Umfang des Rotationsglieds 1, insbesondere an dessen distalem Ende, angeordnet ist. Hierdurch wird während der Drehung des Rotationsglieds 1 relativ zu dem Gehäuse 4 ein akustisches und/oder taktiles Signal erzeugt, an dem erkennbar ist, dass eine Produktausschüttung stattfindet. Ferner wird hierdurch bewirkt, dass das Rotationsglied 1 gegen unerwünschte Drehungen gesichert ist.

Das Gehäuse 4, insbesondere das proximale Ende der Innenhülse 4a bildet das Widerlager für eine schrauben- oder wendelförmige Ausschüttfeder 11, die mit einem Ende an dem Widerlager bzw. dem Gehäuse 4 und mit dem anderen, entgegengesetzten Ende an einem relativ zu dem Gehäuse 4 drehbaren Kupplungsglied 2 befestigt ist. Durch Drehung des Kupplungsglieds 2 relativ zu dem Gehäuse 4 in eine erste Drehrichtung wird die Ausschüttfeder 11 gespannt, wobei die Ausschüttfeder 11 durch Drehung des Kupplungsglieds 2 relativ zu dem Gehäuse 4 in eine zweite, der ersten Drehrichtung entgegengesetzte Drehrichtung entspannt wird. Die gespannte Ausschüttfeder 11 kann mittels der gespeicherten Federenergie das Kupplungsglied 2 in die zweite Drehrichtung drehend antreiben. Die Feder 11 wirkt somit als Dreh- oder Torsionsfeder.

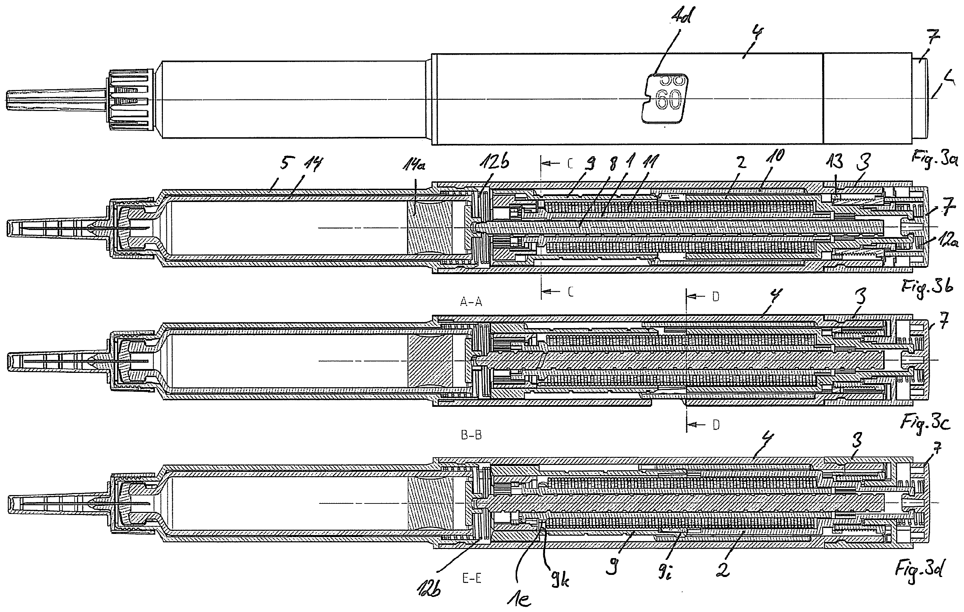

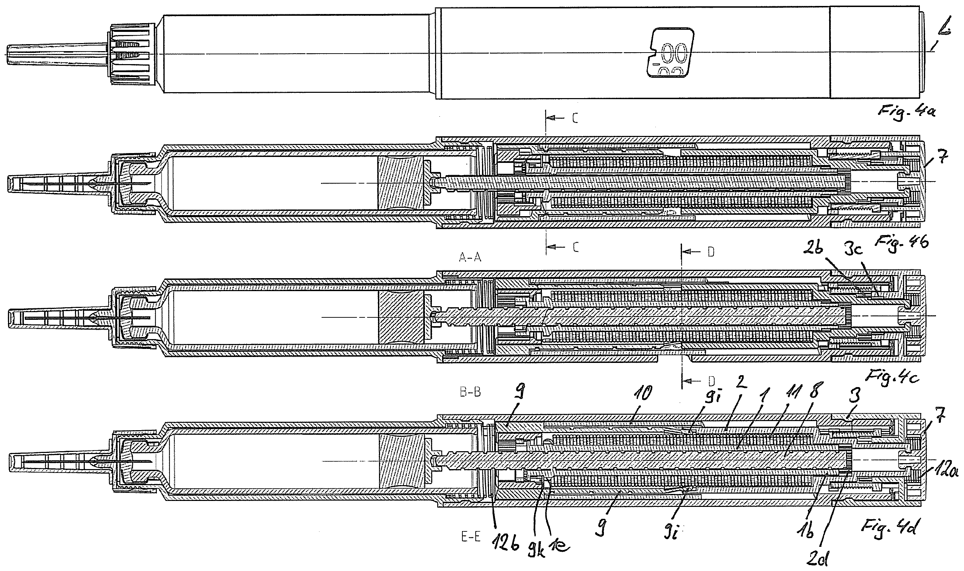

Das Kupplungsglied 2 ist hülsenförmig. Zwischen dem Kupplungsglied 2 und dem Rotationsglied 1 ist eine erste Kupplung 1b, 2d angeordnet, welche das Kupplungsglied 2 und das Rotationsglied 1 drehfest miteinander verbindet, wenn ein am proximalen Ende der Antriebs- und Dosiervorrichtung angeordnetes und als Betätigungsknopf ausgestaltetes Betätigungsglied 7 betätigt ist, d.h. von einem Verwender der Vorrichtung gedrückt ist (Figuren 4a-d). Die Kupplung 1b, 2d entkoppelt das Kupplungsglied 2 und das Rotationsglied 1 voneinander, so dass das Kupplungsglied 2 relativ zu dem Rotationsglied 1 drehbar ist, wenn das Betätigungsglied 7 unbetätigt ist, d.h. von einem Verwender der Vorrichtung nicht gedrückt ist (Figuren 2a-d, 3a-d). Das Betätigungsglied 7 ist axialfest an dem Kupplungsglied 2 befestigt, insbesondere verschnappt. Durch Drücken des Betätigungsglieds 7 wird die erste Kupplung 1b, 2d geschlossen und durch Loslassen des Betätigungsglieds 7 geöffnet. Die erste Kupplung 1b, 2d umfasst eine an dem proximalen Ende des Rotationsglieds 1 gebildete zweite Kupplungsstruktur 1b, die von über den Außenumfang des Rotationsglieds 1 verteilten Zähnen gebildet wird, und eine von dem Kupplungsglied 2 gebildete erste Kupplungsstruktur 2d, die von über den Innenumfang des Kupplungsglieds 2 verteilen Zähnen gebildet wird.

Die Antriebs- und Dosiervorrichtung weist ein drehbar und axialfest an dem Gehäuse 4 befestigtes hülsenförmiges Dosierglied 3 auf, das für eine Dosiserhöhung und/oder ein Vorspannen der Feder 11 in die erste Drehrichtung und für eine Dosisverringerung und/oder ein Entspannen der Feder 11 in die zweite Drehrichtung relativ zu dem Gehäuse 4 gedreht wird.

Zwischen dem Dosierglied 3 und dem Kupplungsglied 2 ist eine zweite Kupplung 2b, 3c angeordnet, welche das Kupplungsglied 2 und das Dosierglied 3 drehfest miteinander verbindet, wenn das Betätigungsglied 7 unbetätigt ist (Figuren 2a-d, 3a-d). Die zweite Kupplung 2b, 3c entkoppelt das Kupplungsglied 2 und Dosierglied 3 voneinander, so dass das Kupplungsglied 2 relativ zu dem Dosierglied 3 drehbar ist, wenn das Betätigungsglied 7 betätigt ist (Figuren 4a-d). Durch Drücken des Betätigungsglieds 7 wird die zweite Kupplung 2b, 3c geöffnet und durch Loslassen des Betätigungsglieds 7 geschlossen. Die zweite Kupplung 2b, 3c umfasst eine von dem Kupplungsglied 2 gebildete dritte Kupplungsstruktur 2b, die von über den Außenumfang des Kupplungsglieds 2 verteilten Zähnen gebildet wird, und eine von dem Dosierglied 3 gebildete vierte Kupplungsstruktur 3c, die von über den Innenumfang des Kupplungsglieds 2 verteilen Zähnen gebildet wird.

Zwischen der vollständig betätigten Position und der unbetätigten Position des Betätigungsglieds 7 kann das Kupplungsglied 2 eine Zwischenposition einnehmen, in der sowohl die Kupplung 2b, 3c als auch die erste Kupplung 1b, 2d eingerückt, d.h. geschlossen sind. Hierdurch wird sichergestellt, dass die zweite Kupplung 2b, 3c erst dann ausgerückt, d.h. geöffnet ist, wenn die erste Kupplung 1b, 2d eingerückt, d.h. geschlossen ist.

Das Rotationsglied 1 weist eine fünfte Kupplungsstruktur 1e in der Gestalt einer über den Umfang angeordneten Außenverzahnung auf. Die fünfte Kupplungsstruktur 1e bildet zusammen mit einer sechsten Kupplungsstruktur 9k, die von dem Lagerelement 9 gebildet wird, eine dritte Kupplung 1e, 9k. Das Bezugszeichen für die sechste Kupplungsstruktur 9k ist an entsprechender Stelle (Querschnittslinie C-C) in die Figuren eingezeichnet, wobei die sechste Kupplungsstruktur 9k jedoch aufgrund der Auswahl der Schnittdarstellungen nicht unmittelbar erkennbar ist. Die sechste Kupplungsstruktur 9k ist an nach innen ragenden flügelartigen Abragungen des Lagerelements 9 gebildet. Die dritte Kupplung 1e, 9k verbindet das Rotationsglied 1 und das Gehäuse 4 und/oder das Lagerelement 9 drehfest miteinander, wenn das Betätigungsglied 7 unbetätigt ist (Figuren 2a-d, 3a-d). Die dritte Kupplung 1e, 9k entkoppelt das Rotationsglied 1 und das Lagerelement 9 voneinander, so dass das Rotationsglied 1 relativ zu dem Gehäuse 4 und dem Lagerelement 9 drehbar ist, wenn das Betätigungsglied 7 betätigt ist (Figuren 4a-d). Durch Drücken des Betätigungsglieds 7 wird die dritte Kupplung 1e, 9k geöffnet und durch Loslassen des Betätigungsglieds 7 geschlossen, wobei das Lagerelement 9 relativ zu dem Rotationsglied 1 entlang der Längsachse L verschoben wird.

Auf das Betätigungsglied 7 wirkt eine als Druckfeder wirkende erste Rücksetzfeder 12a, welche das Betätigungsglied 7 beim Loslassen des Betätigungsglieds 7 in die unbetätigte Position bewegt oder zurücksetzt. Die Rücksetzfeder 12a stützt sich mit einem Ende an dem Dosierglied 3 und dem anderen Ende an dem Betätigungsglied 7 ab. Beim Drücken des Betätigungsglieds 7 wird die Rücksetzfeder 12a gespannt und beim Loslassen entspannt.

Die Ausschüttfeder 11 kann bei der Auslieferung, d. h. im Ausgangszustand der Antriebs- und Dosiervorrichtung (Figuren 2a-d) leicht vorgespannt sein. Durch Drehung des Dosierglieds 3 in die erste Drehrichtung wird die Ausschüttfeder 11 mit soviel Energie vorgespannt, dass die in der Ausschüttfeder 11 gespeicherte Energie ausreicht, die eingestellte Produktdosis vollständig auszuschütten.

Zwischen dem Gehäuse 4 und dem Dosierglied wirkt eine Ratschenfeder 16, welche eine Drehung des Dosierglieds 3 relativ zu dem Gehäuse 4 mit einem Hemmdrehmoment erschwert, wobei das Hemmdrehmoment höher ist als das von der maximal vorgespannten Ausschüttfeder 11 an das Dosierglied 2 übertragene Drehmoment. Hierdurch wird sichergestellt, dass die Ausschüttfeder 11 das Dosierglied 2 beim Loslassen nicht zurückdrehen kann. Die Ratschenfeder 16 bewirkt ferner, dass ein akustisches und/oder taktiles Signal bei der Dosiseinstellung erzeugt wird.

Die vorzugsweise als Stanzteil ausgebildete Ratschenfeder 16 weist wenigstens einen Eingriffsnocken 16a auf, der in eine Stirnverzahnung 4n des Gehäuses 4 eingreift. Die Ratschenfeder 16 vorzugsweise drehfest an dem Dosierglied 3 befestigt. Bei der Drehung des Dosierglieds 3 relativ zu dem Gehäuse 4 rastet der Eingriffsnocken 16a über die Zähne der Stirnverzahnung 4n des Gehäuses 4. Die über den Umfang verteilten Zähne der Verzahnung 4n sind so voneinander beabstandet, dass jeder Rastschritt dosisproportional ist, insbesondere 1 oder 2 IU entspricht.

Durch Drehung des Kupplungsglieds 2 und des damit gekoppelten Rotationsglieds 1 relativ zu dem Gehäuse 4 und dem Vortriebsglied 8 kann die Feder 11 das Vortriebsglied 8 um einen Ausschütthub in distale Richtung verschieben, der proportional zu dem Drehwinkel des Rotationsglieds 1 ist. Durch wahlweises Sperren und Freigeben des Rotationsglieds 1, was durch Betätigen des Betätigungsglieds 7 bewirkt werden kann, kann die Bewegung des Vortriebsglieds 8 relativ zu dem Gehäuse 4, d. h. der Ausschütthub des Vortriebsglieds 8 auf eine vorteilhafte Weise gesteuert werden.

Das Lagerelement 9, welches auch als Anzeigetrommellagerelement bezeichnet werden kann, ist relativ zu dem Gehäuse 4 drehfest aber entlang der Längsachse L verschiebbar angeordnet. Das Lagerelement 9 ist in einem Eingriff mit dem Gehäuse 4, insbesondere im Bereich des Ringspalts zwischen Innenhülse 4a und Außenhülse 4b, der eine Längsbewegung des Lagerelements 9 relativ zu dem Gehäuse 4 zulässt, aber eine Drehbewegung verhindert. Der Eingriff kann durch eine Längsführung zwischen dem Lagerelement 9 und dem Gehäuse 4 gebildet werden.

Das Lagerelement 9 weist ein Gewinde 9a, insbesondere ein Außengewinde auf, in welches ein Gewinde 10e, insbesondere ein Innengewinde, des Dosisanzeigeelements 10 eingreift. Durch diesen Gewindeeingriff ist das Anzeigeelement 10 relativ zu dem Lagerelement 9 schraubbar.

Das Dosisanzeigeelement 10 ist drehfest, aber axial verschiebbar mit dem Kupplungsglied 2 verbunden, insbesondere in einem Eingriff. Dieser Eingriff umfasst eine Längsführung 2a, welche bewirkt, dass das Dosisanzeigeelement 10 relativ zu dem Kupplungsglied 2 drehfest, aber axial verschiebbar ist. Eine Drehung des Kupplungsglieds 2 relativ zu dem Lagerelement 9 bewirkt aufgrund der drehfesten Verbindung zwischen Kupplungsglied 2 und Dosisanzeigeelement 10, dass das Dosisanzeigeelement 10 ebenfalls gedreht und aufgrund des Gewindeeingriffs in das Gewinde 9a an dem Lagerelement 9 entlang geschraubt wird, insbesondere zusätzlich zu dem aufgrund des Eingriffsnockens 16a erzeugten Klickgeräusch.

Das Dosisanzeigeelement 10 weist über seinen Außenumfang eine sich entsprechend der Steigung des Gewindes 10e wendelförmig ersteckende Dosisskala 10b auf, die eine Vielzahl nacheinander angeordnete Skalenwerte umfasst. In dem gezeigten Beispiel kann mit der Antriebs- und Dosiervorrichtung eine maximale Dosis von 60 IU eingestellt werden, wobei die Skala von 0 bis 60 reicht und die Dosiswerte in Zweierschritten angegeben sind.

Ebenfalls entsprechend der Steigung des Gewindes 10e ist eine Markierung 10a wendelförmig über den Außenumfang des Dosisanzeigeelements 10 angeordnet. Diese Markierung 10a dient - wie weiter unten beschrieben wird - zur Anzeige, ob die Vorrichtung bzw. das Betätigungsglied 7 betätigt oder unbetätigt ist. Die Markierung 10a ist eine optionale Einrichtung. Sie kann sich entlang der ganzen Dosisskala 10b oder nur Teilen oder einem einzelnen Skalenwert davon erstrecken. Insbesondere kann sie bei betätigter Antriebs- und Dosiervorrichtung nur gegen Ende der Produktausschüttung oder in der Nulldosisposition sichtbar sein.

Das Dosisanzeigeelement 10 weist an seinem z.B. distalen Ende eine in Umfangsrichtung weisende und wirkende Anschlagfläche 10c auf, die als Nulldosisanschlag bezeichnet wird. Das Dosisanzeigeelement 10 weist an seinem, z.B. dem distalen Ende entgegengesetzten, proximalen Ende eine in Umfangsrichtung weisende und wirkende Anschlagfläche 10d auf, welche als Maximaldosisanschlag bezeichnet wird.

Das Dosisanzeigelement 10 ist an dem Lagerelement 9 zwischen einer Nulldosisposition und einer Maximaldosisposition hin und her schraubbar. In der Maximaldosisposition verhindert der Maximaldosisanschlag 10d im Zusammenwirken mit einem in diesem Beispiel von dem Gehäuse 4 gebildeten Maximaldosisgegenanschlag 4f die Drehung des Dosisanzeigeelements 10 in eine erste Drehrichtung, nämlich eine Drehrichtung, welche eine Erhöhung der Dosis über den maximal einstellbaren Wert hinaus bewirken würde. In dieser Maximaldosisposition ist das Dosisanzeigeelement 10 in die entgegengesetzte, d. h. zweite Drehrichtung drehbar.

In der Nulldosisposition, die z. B. in Figur 2b gezeigt wird, verhindert der Nulldosisanschlag 10c im Zusammenwirken mit einem Nulldosisgegenanschlag 9c, der von dem Lagerelement 9 gebildet wird, die Drehung des Dosisanzeigeelements 10 in die zweite Drehrichtung, welche bewirken würde, dass eine kleinere Dosis als Null eingestellt wird. Die Drehung in die erste Drehrichtung ist in der Nulldosisposition möglich. Obgleich der Nulldosisgegenanschlag 9c von dem Lagerelement 9 gebildet wird, kann dieser abweichend von diesem Beispiel optional von dem Gehäuse 4 gebildet werden. Der Maximaldosisgegenanschlag 4d kann abweichend von dem gezeigten Beispiel von dem Lagerelement 9 oder einem anderen Teil gebildet werden, das aber vorzugsweise drehfest mit dem Gehäuse 4 verbunden ist.

Das Gehäuse 4 weist eine Zeigeeinrichtung 4d in der Gestalt eines Fensters auf, welches den Blick auf die Skala 10b des Dosisanzeigeelements 10 frei gibt. Das Gehäuse 4 weist eine Ringnut 4g auf, in welche insbesondere eine Ringschulter des Dosierglieds 3 für eine drehbare und axialfeste Verbindung eingreift. Das Dosierglied 3 kann über seinen Außenumfang eine Griffstruktur aufweisen, welche es dem Verwender der Vorrichtung erleichtert, das Dosierglied 3 relativ zu dem Gehäuse 4 zu verdrehen. Im unbetätigten Zustand des Betätigungsglieds bewirkt eine Drehung des Dosierglieds 3 neben der Änderung der Spannung der Ausschüttfeder 11 eine Drehung oder Schraubbewegung des Dosisanzeigeelements 10, wodurch die gewünschte Dosis einstellbar und in der Zeigeeinrichtung 4d ablesbar ist.

Das Betätigungsglied 7 ist relativ zu dem Dosierglied 3 entlang der Längsachse L für eine Betätigung der Vorrichtung zur Produktausschüttung bewegbar. Das Betätigungsglied 7 bildet das proximale Ende der Vorrichtung und ist vom Daumen der Hand des Verwenders, welche das Gehäuse 4 umgreift, auf einfache Weise betätigbar, insbesondere relativ zu dem Gehäuse 4 und/oder zu dem Dosierglied 3 verschiebbar.

Das Kupplungsglied 2 stößt insbesondere mit seiner distalen Stirnseite an das Lagerelement 9, insbesondere an einem am proximalen Ende des Lagerelements 9 federnd angeordneten Schnapper 9i an, wodurch das Lagerelement 9 während der Betätigung des Betätigungsglieds 7, die eine Verschiebung Kupplungsglieds 2 in distale Richtung bewirkt, von dem Kupplungsglied 2 mitgenommen, d.h. ebenfalls in distale Richtung verschoben wird. Die Antriebs- und Dosiervorrichtung weist ferner eine weitere Rücksetzfeder 12b auf, die beim Betätigen des Betätigungsglieds 7 gespannt wird und die bewirkt, dass das Lagerelement 9 zurücksetzt oder in proximale Richtung verschoben wird. Die zweite Rücksetzfeder 12b stützt sich mit ihrem distalen Ende vorzugsweise an der Produktbehälteraufnahme 5 oder dem Produktbehälter 14 und mit ihrem proximalen Ende vorzugsweise an dem Lagerelement 9 ab. Die Rücksetzfedern 12a, 12b sind vorzugsweise als Schrauben- oder Wendelfeder ausgestaltet, welche als Druckfeder wirkt.

An insbesondere dem Innenumfang des Dosisanzeigeelements 10 kann eine Erhöhung oder ein Nocken gebildet sein, die oder der so angeordnet ist, dass sie oder er bei der Drehung des Dosisanzeigeelements in die zweite Drehrichtung, d.h. beim Herunterzählen der Dosiswerte in der Zeigeeinrichtung 4d, kurz bevor die Dosis 0 erreicht wird oder der Nulldosisanschlag 10c in den Eingriff mit dem Nulldosisgegenanschlag 9c gelangt, insbesondere bei Durchlaufen der Dosis 1 IU oder 2 IU oder allgemeiner schon beim Durchlaufen eines Werts kleiner 6 IU, den Schnapper 9i auslenkt, insbesondere nach innen und/oder aus dem anstoßenden Eingriff mit dem Kupplungsglied 2 auslenkt, wodurch das Kupplungsglied 2 nicht mehr an den Schnapper 9i anstößt. Hierdurch wird bewirkt, dass das Lagerelement trotz betätigtem Betätigungsglied 7 in proximale Richtung bewegbar und/oder an der Anstoßstelle, an welcher das Kupplungsglied 2 an dem Schnapper 9i angestoßen ist, vorbeibewegt wird, insbesondere mit einer axialen Bewegung in proximale Richtung. Durch die zweite Rücksetzfeder 12b wird das Lagerelement 9 schlagartig in proximale Richtung verschoben oder beschleunigt, wobei diese Bewegung des Lagerelements 9 durch einen von dem Gehäuse 1 gebildeten Anschlag gestoppt wird. Das Anschlagen an dem Anschlag bewirkt ein akustisches oder/taktiles Signal, insbesondere ein Klickgeräusch, welches dem Verwender anzeigt, dass die Ausschüttung der Dosis beendet oder nahezu beendet ist. Das Lagerelement 9 weist für den Anschlag einen Gegenanschlag auf, der von einem Vorsprung gebildet wird, der zusätzlich die Axialbewegung in proximale Richtung begrenzt.

Das Dosierglied 3 ist relativ zu dem Betätigungsglied 7 drehfest. Das Betätigungsglied 7 durchgreift eine nach innen ragende Schulter des Dosierglieds 3. Zwischen dem distalen Ende des Betätigungsglieds 7 und einer Stufe des Kupplungsglieds 2 ist eine Hülse mit einem Außengewinde 3d axialfest eingefasst, wobei diese Hülse drehfest mit dem Dosierglied 3 verbunden ist.

Die Antriebs- und Dosiervorrichtung weist einen Dosisbegrenzer 13 in der Gestalt eines Rings, eines Ringsegments oder einer Mutter auf, der an seinem Innenumfang ein Gewinde 13b aufweist, der in das Gewinde 3d eingreift, so dass der Begrenzer 13 relativ zu dem dem Dosierglied 3 schraubbar ist. Am Außenumfang weist der Begrenzer 13 ein Eingriffsglied 13a auf, welches in eine Längsführung am Innenumfang des Gehäuses 4 eingreift, so dass das der Dosisbegrenzer 13 relativ zu dem Gehäuse 4 drehfest aber axial verschiebbar ist. An dem Dosierglied 3 oder der das Gewinde 3d aufweisende Hülse ist ein Stoppanschlag gebildet, von dem der Begrenzer 13 proportional zu der maximal aus dem Produktbehälter 14 ausschüttbaren Produktmenge beabstandet ist. Da bei der Dosiseinstellung das Dosierglied 3 relativ zu dem Gehäuse 4 verdreht und bei einer Dosisausschüttung nicht verdreht wird, kann durch den Begrenzer 13 ein Zählwerk gebildet werden, welches die bereits ausgeschütteten Einzeldosen und die aktuell eingestellte Dosis addiert und sich dementsprechend immer näher an den Stoppanschlag annähert. Eine Dosiserhöhung bewirkt, dass der Begrenzer 13 zu dem Stoppanschlag hin bewegt wird. Eine Dosisverringerung bewirkt, dass der Begrenzer 13 von dem Stoppanschlag weg bewegt wird. Ist die in dem Produktbehälter 14 angegebene Restdosis geringer als die maximal mit der Antriebs- und Dosiervorrichtung einstellbare Dosis, gerät der Begrenzer 13 in einen Kontakt mit dem Stoppanschlag, so dass eine Verdrehung des Dosierglieds 3 relativ zu dem Gehäuse 4 in eine Drehrichtung, die eine Erhöhung der Dosis zur Folge hätte (erste Drehrichtung), blockiert wird.

Insbesondere dadurch, dass die zweite Kupplung 2b, 3c und die dritte Kupplung 1e, 9k geöffnet sind, kann sich die Ausschüttfeder 11 entspannen, wobei das Kupplungsglied 2 relativ zu dem Gehäuse 4 verdreht wird. Aufgrund der geschlossenen Kupplung ersten 1b, 2d wird das Rotationsglied 1 mit dem Kupplungsglied 2 mitgedreht. Aufgrund des drehfesten Eingriffs zwischen dem Kupplungsglied 2 und dem Dosisanzeigeelement 10 wird das Dosisanzeigeelement 10 ebenfalls mit dem Kupplungsglied 2 mitgedreht, wodurch das Dosisanzeigeelement 10 in seine Nulldosisposition zurück geschraubt und das Vortriebsglied 8 proportional zu dem sich insbesondere in Umfangsrichtung erstreckenden Abstand zwischen Nulldosisanschlag 10c und Nulldosisgegenanschlag 9c um einen Ausschütthub relativ zu dem Gehäuse 4 in distale Richtung verschoben wird. Die Drehung des Rotationsglieds 1 relativ zu dem Gehäuse 4 bewirkt, dass das Rastglied 1d über die Verzahnung 4m insbesondere in dosisproportionalen Winkelschritten rastet und dabei das akustische und /oder taktile Signal erzeugt.

In den Figuren 2a bis 2d wird die Antriebs- und Dosiervorrichtung, die auch als Injektionsvorrichtung bezeichnet werden kann, in ihrem Ausgangs- oder Auslieferungszustand, insbesondere vor einer ersten Verwendung gezeigt. Die in der Zeigeeinrichtung 4d angezeigte Produktdosis ist 0. Ein Betätigen des Betätigungsglieds 7 hätte zur Folge, dass keine Dosis ausgeschüttet wird. Der Begrenzer 13 weist einen Abstand zu dem Stoppanschlag auf, welcher proportional zu der in dem Produktbehälter 14 enthaltenen oder ausschüttbaren Produktmenge ist, wie z. B. 300 IU

Zur Einstellung der Produktdosis wird das Dosiseinstellglied 3 relativ zu dem Gehäuse 4 verdreht, wodurch aufgrund der geschlossenen zweiten Kupplung 2b, 3c das Kupplungsglied 2 und somit auch das Dosisanzeigeelement 10 relativ zu dem Gehäuse 4 verdreht werden. Dabei schraubt sich das Dosisanzeigeelement 10 entlang dem Lagerelement 9 aufgrund des Gewindeeingriffs des Gewindes 10e mit dem Gewinde 9a. Die geöffnete erste Kupplung 1b, 2d und die geschlossene dritte Kupplung 1e, 9k verhindern, dass das Rotationsglied 1 mit dem Dosiseinstellglied 3 mitgedreht wird. Insbesondere wird der Abstand zwischen dem Nulldosisanschlag 10c und dem Nulldosisgegenanschlag 9c proportional zu der in der Zeigeeinrichtung 4d gezeigten Dosis erhöht. Bei der Drehung wird aufgrund des Überrastens des Eingriffsnockens 16a über die Stirnverzahnung 4n ein hörbares und fühlbares Signal erzeugt. Schließlich wird durch das sich in die erste Drehrichtung drehende Kupplungsglied 2 die Ausschüttfeder 11 gespannt und mit der für die Produktausschüttung notwendigen Energie versorgt.

In den Figuren 3a bis 3d wird die Antriebs- und Dosiervorrichtung in einem Zustand gezeigt, in der eine maximal einstellbare Dosis eingestellt wurde, nämlich in diesem Beispiel 60 IU, die in der Zeigeeinrichtung 4d ablesbar sind. Eine weitere Dosiserhöhung ist aufgrund des Zusammenwirkens, insbesondere des Kontakts des Maximaldosisanschlags 10d mit dem Maximaldosisgegenanschlag 4f nicht möglich. Der Dosisbegrenzer 13 ist, wie am besten aus den Figuren 3b bis 3d erkennbar ist, entsprechend 60 IU weit an den Stoppanschlag herangerückt oder verschoben.

Zur Ausschüttung der z. B. in Figur 3a angezeigten Dosis wird das Betätigungsglied 7 betätigt, insbesondere gedrückt, d. h. relativ zu dem Gehäuse 4 und dem Dosierglied 3 in distale Richtung verschoben, wodurch das Kupplungsglied 2 und das Lagerelement 9 sowie das Dosisanzeigeelement 10 relativ zu dem Gehäuse 4 in distale Richtung verschoben werden, insbesondere gegen die Kraft der Kupplungs- oder Rücksetzfeder 12. Dadurch, dass das Dosisanzeigeelement 10 relativ zu dem Gehäuse 4 und der Zeigeeinrichtung 4d axial verschoben wird, erscheint die in Figur 1 gezeigte Markierung 10a in der Zeigeeinrichtung 4d, wodurch der Verwender optisch ablesen kann, dass die Vorrichtung betätigt ist. Durch die Verschiebung des Dosisanzeigeelements 10 relativ zu dem Gehäuse 4 und der Zeigeeinrichtung 4d wird die Markierung 10a aus einer Position, in der sie von dem Gehäuse 4 verdeckt wird, in eine Position, in der sie in der Zeigeeinrichtung 14d gezeigt wird, entlang der Längsachse L verschoben.

Die Betätigung des Betätigungsglieds 7 bewirkt ferner, dass die erste Kupplungsstruktur 2d in die zweite Kupplungsstruktur 1b einrückt, d.h. ein Schließen der ersten Kupplung 1b, 2d, dass die dritte Kupplungsstruktur 2b aus der vierten Kupplungsstruktur 3c ausrückt, d.h. ein Öffnen der zweiten Kupplung 2b, 3c, und dass sechste Kupplungsstruktur 9k aus der fünften Kupplungsstruktur 1e ausrückt, d.h. ein Öffnen der dritten Kupplung 1e, 9k, so dass das Kupplungsglied 2 in Bezug auf das Gehäuse 4 nicht mehr drehfest sondern drehbar ist. Im betätigten Zustand des Betätigungsglieds 7 sind das Rotationsglied 1, das Kupplungsglied 2 und das Dosisanzeigeelement 10 verdrehfest miteinander verbunden, wodurch sich das Rotationsglied 1, das Kupplungsglied 2 und das Dosisanzeigeelement 10 gemeinsam relativ zu dem Gehäuse 4 drehen können. Durch das Drehmoment der in der Ausschüttfeder 11 gespeicherten Energie auf das Kupplungsglied 2 werden einerseits das Dosisanzeigeelement 10 an dem Lagerelement 9 in Richtung Nulldosisposition zurückgeschraubt, wobei die in der Zeigeeinrichtung 14d angezeigte Dosis runterzählt, und andererseits das Vortriebsglied 8 vermittelt durch das Rotationsglied 1 relativ zu dem Gehäuse 4 in distale Richtung um den Ausschütthub bewegt, welcher proportional zu der zuvor eingestellten Dosis ist.

Kurz bevor das Dosisanzeigeelement 10 seine Nulldosisposition erreicht hat wird der Schnapper 9i mittels des Dosisanzeigeelements 10 ausgelenkt, wodurch das Lagerelement 9 mittels der zweiten Rücksetzfeder 12b relativ zu dem Kupplungsglied 2 in proximale Richtung verschoben wird, wodurch kurz vor oder beim Erreichen der Nulldosisposition ein für den Verwender fühl- und hörbares Signal erzeugt wird.

Wenn das Dosisanzeigeelement 10 seine Nulldosisposition erreicht hat (Figuren 4a bis 4d) ist die zuvor eingestellte Dosis oder Einzeldosis ausgeschüttet. Lässt der Verwender das in den Figuren 4a bis 4c noch als gedrückt dargestellte Betätigungsglied 7 los, setzt die erste Kupplungs- oder Rücksetzfeder 12a das Betätigungsglied 7 und das Kupplungsglied 2 in die z. B. in den Figuren 2a-d gezeigte Position zurück. Beim Zurücksetzen wird das Kupplungsglied 2 relativ zu dem Lagerelement 9 in proximale Richtung verschoben, wodurch der Schnapper 9i in die Position zurückfedern kann, in welcher das Kupplungsglied 2 an dem Schnapper 9i anstoßen kann. Beim Rücksetzen werden die genannten Elemente relativ zu dem Gehäuse 4 oder dem Dosierglied 3 in proximale Richtung verschoben.

Beim Zurücksetzen der Vorrichtung mittels der ersten und/oder zweiten Feder 12a, 12b werden die erste Kupplungsstruktur 2d von der zweiten Kupplungsstruktur 1b ausgerückt und die dritte Kupplungsstruktur 2b in die vierte Kupplungsstruktur 3c und die sechste Kupplungsstruktur 9k in die fünfte Kupplungsstruktur 1e eingerückt. Das Kupplungsglied 2 ist nun wieder drehfest in Bezug auf das Dosierglied 3, wobei das Dosierglied 3 zusammen mit dem Dosisanzeigeelement 10 relativ zu dem Gehäuse 4 und/oder der Zeigeeinrichtung 4d und/oder dem Rotationsglied 1 für eine neue Einstellung einer Produktdosis oder Einzeldosis drehbar ist.

Nach mehreren so erfolgten Ausschüttungen nimmt der Begrenzer 13 seine Stoppposition ein (nicht gezeigt), d. h. er schlägt dem Stoppanschlag an, wodurch der Begrenzer 13 eine Dosiseinstellung auf einen Wert, welcher die in dem Produktbehälter 14 enthaltene Restmenge übersteigt, blockiert. Sind z.B. nur noch 56 IU in dem Produktbehälter 14 enthalten, wobei mit der Antriebs- und Dosiervorrichtung maximal 60 IU einstellbar wären, kommt der Begrenzer 13 bereits bei 56 IU in einen Kontakt mit dem Stoppanschlag, so dass das Dosierglied 3 für eine Drehung in die erste Richtung, welche eine Erhöhung der Dosis bewirken würde, gesperrt wird. Die Verringerung der Dosis ist jedoch durch Drehen des Dosierglieds 3 in die zweite Drehrichtung möglich.

Nach z.B. vollständiger Entleerung des Produktbehälters 14, wird die Antriebs- und Dosiervorrichtung oder Injektionsvorrichtung als ganzes entsorgt. Es handelt sich somit um eine Einweg-Injektionsvorrichtung. Grundsätzlich können die hierin gezeigten Antriebs- und Dosiervorrichtungen aber auch in Verbindung mit Mehrweginjektionsvorrichtungen Anwendung finden, bei denen ein entleerter Produktbehälter 14 gegen einen neuen ausgetauscht wird.

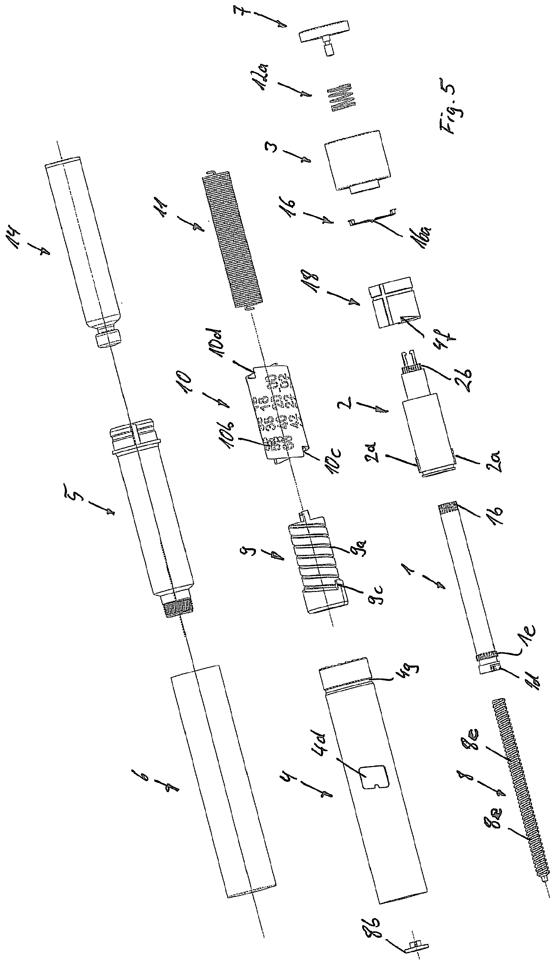

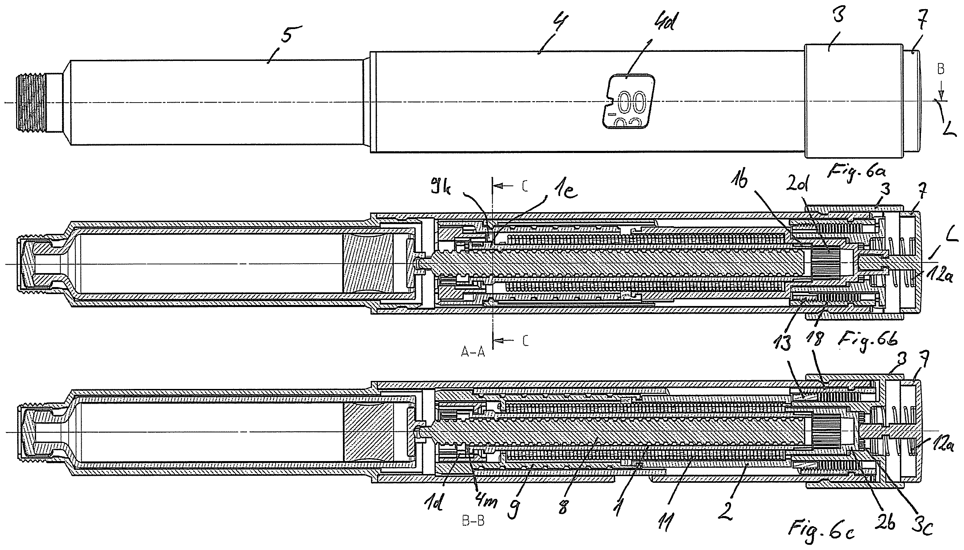

Eine zweite Ausführungsform einer Antriebs - und Dosiervorrichtung wird in den Figuren 5 bis 6c dargestellt. Im Folgenden werden die Merkmale beschrieben, mit denen sich diese Ausführungsform von der ersten unterscheidet, so dass im Übrigen auf die Beschreibung zu den Figuren 1 bis 4d verwiesen wird. Gleiche Bezugszeichen bezeichnen zumindest funktionell gleichartige Teile.

Die Antriebs- und Dosiervorrichtung unterscheidet sich insbesondere dadurch von der Ausführungsform aus den Figuren 1 bis 4d, dass das Kupplungsglied 2 in solch einem Eingriff mit dem Lagerelement 9 ist, dass das Kupplungsglied 2 relativ zu dem Lagerelement 9 drehbar und axialfest ist. Hierdurch können die Schnapper 9i eingespart werden, da nicht immer ein Signal erforderlich ist, welches während der Produktausschüttung das Erreichen der Nulldosisposition signalisiert. Ferner kann hierdurch die zweite Rücksetzfeder 12b eingespart werden, da die erste Rücksetzfeder 12a das Kupplungsglied 2 zurücksetzt und das Kupplungsglied 2 aufgrund des Eingriffs mit dem Lagerelement 9 das Lagerelement 9 mitnimmt.

Ferner ist der Maximaldosisgegenanschlag 4f nicht an der äußeren Gehäusehülse 4b, sondern an einer dreh- und vorzugsweise auch axialfest mit der Gehäusehülse 4b verbundenen Hülse 18 gebildet. Die Hülse 18 kann nicht zuletzt wegen ihres dreh- und axialfesten Eingriffs mit der äußeren Gehäusehülse 4b als Teil des Gehäuses 4 angesehen werden.

Die Hülse 18 bildet insbesondere alternativ oder zusätzlich ein Innengewinde 4l, in welches ein Außengewinde 13b des Begrenzers 13 eingreift, wobei der Begrenzer 13 in einem drehfesten aber axial verschiebbaren Eingriff mit dem Dosierglied 3 ist.

Der Begrenzer 13 ist somit relativ zu dem Gehäuse 4 oder der Hülse 18 schraubbar. Am Innenumfang weist der Begrenzer 13 ein Eingriffsglied auf, welches in eine Längsführung des Dosierglieds 3 eingreift. An dem Dosierglied 3 oder der das Gewinde 3d aufweisende Hülse ist ein Stoppanschlag in der Gestalt eines Axialanschlags gebildet, von dem der Begrenzer 13 proportional zu der maximal aus dem Produktbehälter 14 ausschüttbaren Produktmenge beabstandet ist. Hierdurch wird das in der ersten Ausführungsform beschriebene Zählwerk gebildet auf das hiermit verwiesen wird.

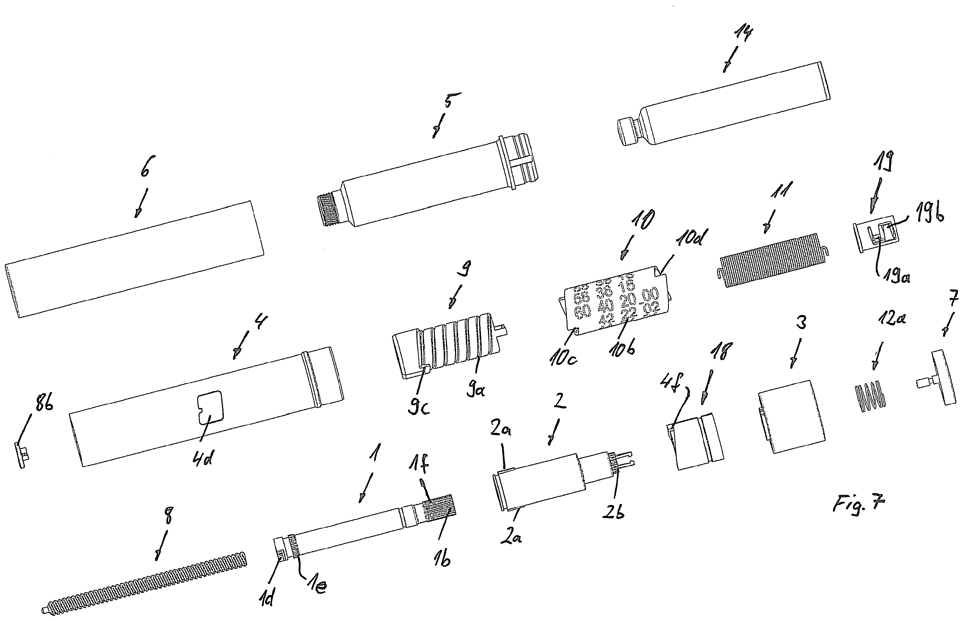

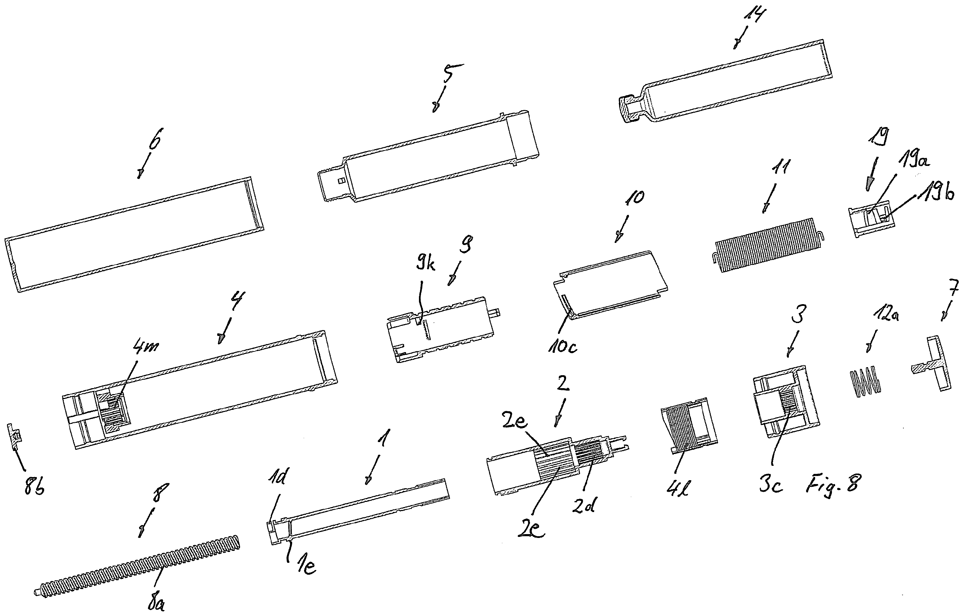

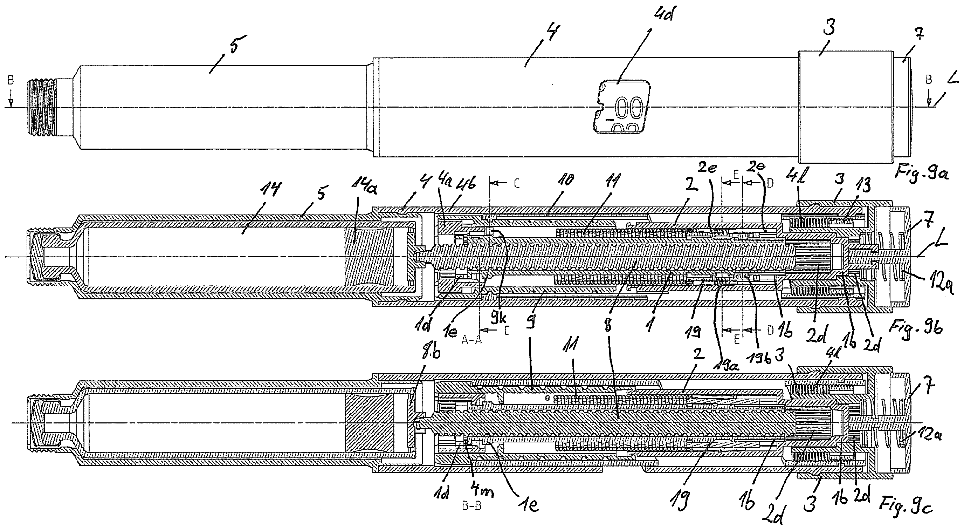

Eine dritte Ausführungsform einer Antriebs - und Dosiervorrichtung wird in den Figuren 7 bis 9c dargestellt. Im Folgenden werden die Merkmale beschrieben, mit denen sich diese Ausführungsform von der zweiten Ausführungsform unterscheidet, so dass im Übrigen auf die Beschreibung zu den Figuren 1 bis 6d verwiesen wird. Gleiche Bezugszeichen bezeichnen zumindest funktionell gleichartige Teile.

Die Antriebs- und Dosiervorrichtung unterscheidet sich insbesondere dadurch von der zweiten Ausführungsform, dass die Ausschüttfeder 11 bei der Drehung des Dosierglieds 3 in die erste Drehrichtung, d.h. bei Dosiserhöhung, gespannt und in die zweite Drehrichtung, d.h. bei Dosisverringerung, nicht entspannt wird. Hierzu ist ein Ratschenelement 19, das auch als Ratschenhülse bezeichnet werden kann, vorgesehen, das kinematisch zwischen der Ausschüttfeder 11 und dem Dosierglied 3 angeordnet ist und eine Drehung des Dosierglieds in die erste Drehrichtung an die Ausschüttfeder 11 übertragt, so dass die Ausschüttfeder 11 gespannt wird, und eine Drehung in die zweite Drehrichtung an die Ausschüttfeder 11 nicht überträgt, d.h. die Dosierhülse 2 von der Antriebsfeder 11 rotatorisch entkoppelt, so dass die Ausschüttfeder 11 nicht entspannt wird.

Das proximale Ende der Ausschüttfeder 11 ist verdrehgesichert an dem hülsenförmigen Ratschenelement 19 befestigt. Das Ratschenelement 19 weist ein erstes federnd angeordnetes Eingriffselement 19a auf, welches in eine Innenverzahnung 2e des Kupplungsglieds 2 eingreift, wobei das Eingriffselement 19a so mit der Innenverzahnung 2e zusammenwirkt, dass das Ratschenelement 19 bei Drehung des Kupplungsglieds 2 in die erste Drehrichtung mit dem Kupplungsglied 2 mitgedreht wird, wobei sich das Kupplungsglied 2 bei Drehung in die zweite Drehrichtung relativ zu dem Ratschenelement 19 dreht. Die Innenverzahnung weist vorzugsweise sägezahnförmige Zähne auf, die jeweils eine flache und eine steile Flanke aufweisen, wodurch diese Wirkung erzielt wird.

Das Ratschenelement 19 weist ein zweites federnd angeordnetes Eingriffselement 19b auf, welches in eine Außenverzahnung 1f des Rotationsglieds 1 eingreift, wobei das zweite Eingriffsglied 19b so mit der Außenverzahnung 1f zusammenwirkt, dass eine Drehung des Ratschenelements 19 relativ zu dem Kupplungsglied 2 in die erste Drehrichtung möglich ist und in die entgegengesetzte Drehrichtung nicht möglich ist, d.h. gesperrt ist. Wird eine Dosisverringerung vorgenommen, dreht sich das Kupplungsglied 2 relativ zu dem Ratschenelement 19, wobei das erste Eingriffsglied 19a über die Innenverzahnung 2e rastet und sich das zweite Eingriffsglied 19b drehfest an der Außenverzahnung 1f des Rotationsglieds 1 abstützt. Die geschlossene dritte Kupplung 1e, 9k verhindert, dass das Rotationsglied 1 sich in die zweite Drehrichtung dreht. In dem gezeigten Beispiel bilden die zweite Kupplungsstruktur 1b und die Außenverzahnung 1f eine gemeinsame Verzahnung, wobei sie selbstverständlich auch voneinander getrennt sein könnten.

Wie für die anderen Ausführungsformen beschrieben, wird für die Ausschüttung die dritte Kupplung 1e, 9k geöffnet, wodurch die Ausschüttfeder 11 das Rotationsglied 1 in die zweite Drehrichtung antreibt.

Die Innenverzahnung 1b und das erste Eingriffselement 19a sind so aufeinander abgestimmt, insbesondere durch Anpassung der steilen Flanke der Zähne der Innenverzahnung 1b, dass das Kupplungsglied 2 bei Drehung in die erste Drehrichtung das Ratschenelement 19 mitnimmt, sofern ein gewisses Grenzdrehmoment unterschritten wird und relativ zu dem Ratschenelement 19 verdreht wird, wenn das Grenzdrehmoment erreicht oder überschritten wird. Das Grenzdrehmoment ist so gewählt, dass die Ausschüttfeder nicht über den linearen Bereich der Federkonstante hinaus gespannt werden kann, wodurch vorteilhaft eine Beschädigung der Ausschüttfeder 11 durch übermäßiges Spannen verhindert wird. Das Zusammenwirken des Ratschenelements 19 mit dem Kupplungsglied 2 kann somit nach dem Prinzip einer drehmomentbegrenzenden Rutschkupplung erfolgen.

**Bezugszeichenliste**

| | | | |
|---|---|---|---|
| 1 | Rotationsglied / Gewindestange / Gewindemutter | 9i | Schnapper |
| | | 9k | sechste Kupplungsstruktur |
| 1a | Innengewinde | | |
| 1b | zweite Kupplungsstruktur / Zähne | 10 | Dosisanzeigeelement / Dosisanzeigetrommel |
| 1d | Rastglied | | |
| 1e | fünfte Kupplungsstruktur | 10a | Markierung |
| 1f | Außenverzahnung | 10b | Dosisskala |
| | | 10c | Nulldosisanschlag |
| 2 | Kupplungsglied / Anzeigekupplung | 10d | Maximaldosisanschlag |
| 2a | Längsführung / Längsrippe | 10e | Innengewinde |
| 2b | dritte Kupplungsstruktur | | |
| 2c | Ringnut | 11 | Feder / Ausschüttfeder |
| 2d | erste Kupplungsstruktur | | |
| 2e | Innenverzahnung | 12a | erste Feder / Rücksetz- oder Kupplungsfeder |
| 3 | Dosierglied / Dosierknopf / Dosiseinstellglied | 12b | zweite Feder / Rücksetz- oder Kupplungsfeder |
| 3c | vierte Kupplungsstruktur | | |
| 3d | Außengewinde | 13 | Begrenzer / Dosisbegrenzer |
| | | 13a | Eingriffsglied |
| 4 | Gehäuse | 13b | Gewinde / Innengewinde / |
| 4a | Innenhülse | | Außengewinde |
| 4b | Außenhülse | | |
| 4d | Zeigeeinrichtung / Fenster | 14 | Produktbehälter / Karpule |
| 4f | Maximaldosisgegenanschlag | 14a | Kolben |
| 4g | Ringnut | 14b | Septum |
| 4l | Gewinde / Innengewinde | | |
| 4m | Innenverzahnung | 15 | Schalthülse |
| 4n | Stirnverzahnung | | |
| | | 16 | Ratschenfeder |
| 5 | Produktbehälteraufnahme | 16a | Eingriffsnocken 16a |
| 6 | Schutzkappe | 17 | Nadeleinheit |
| 7 | Betätigungsglied / Betätigungsknopf | 18 | Hülse |
| 8 | Vortriebsglied / Stößel | 19 | Ratschenelement / Ratschenhülse |
| 8a | Längsführung | 19a | erstes Eingriffselement |
| 8b | Flansch | 19b | zweites Eingriffselement |
| 8c | Gewinde / Außengewinde | | |
| 8e | Gewindestange | 1b, 2d | erste Kupplung / Ausschüttkupplung |
| | | 2b, 3c | zweite Kupplung / Dosierkupplung |
| 9 | Lagerelement | 1e, 9k | dritte Kupplung |
| 9a | Außengewinde | | |
| 9c | Nulldosisgegenanschlag | L | Längsachse / Drehachse |

## Patentansprüche

1. Antriebs- und Dosiervorrichtung für eine Injektionsvorrichtung zur Verabreichung eines flüssigen Produkts, wobei mit der Antriebs- und Dosiervorrichtung eine zu verabreichende Produktdosis einstellbar ist, umfassend:
a) ein Gehäuse (4),
b) ein Dosisanzeigeelement (10), über dessen Umfang eine Dosisskala (10b) angeordnet ist,
c) eine Zeigeeinrichtung (4d) und ein vom Verwender der Antriebs- und Dosiervorrichtung greifbares Dosierglied (3), wobei zur Einstellung der zu verabreichenden Dosis durch Drehung des Dosierglieds (3) relativ zu der Zeigeeinrichtung (4d) das Dosisanzeigeelement (10) relativ zu der Zeigeeinrichtung (4d) und um eine Drehachse (L) drehbar ist und mittels der Zeigeeinrichtung (4d) ein Wert der Dosisskala (10b) ablesbar ist, welcher der eingestellten Dosis entspricht,
d) eine Ausschüttfeder (11), welche die für die Ausschüttung des Produkts erforderliche Energie speichert, wobei die Ausschüttfeder (11) mittels Drehung des Dosierglieds (3) gespannt wird oder spannbar ist,
e) ein vom Verwender der Antriebs- und Dosiervorrichtung betätigbares Betätigungsglied (7) zur Ausschüttung der eingestellten Produktdosis,
wobei
f) die Antriebs- und Dosiervorrichtung eine Kupplung (2b, 3c) umfasst, die das Dosierglied (3) so mit der Ausschüttfeder (11) verbindet, dass bei unbetätigtem Betätigungsglied (7) die Ausschüttfeder (11) mittels Drehung des Dosierglieds (3) spannbar ist, und dass durch Betätigung des Betätigungsglieds (7) das Dosierglied (3) von der Ausschüttfeder (11) entkoppelt wird
**gekennzeichnet durch** ein Lagerelement (9), mit dem das Dosisanzeigeelement (10) in einem Eingriff ist, der die Dreh- oder Schraubbewegung des Dosisanzeigeelements (10) relativ zu der Zeigeeinrichtung (4d) bewirkt, wobei das Lagerelement (9) zusammen mit dem Dosisanzeigeelement (10) relativ zu dem Gehäuse (4) und entlang der Drehachse (L) verschiebbar ist.

2. Antriebs- und Dosiervorrichtung nach Anspruch 1, **dadurch gekennzeichnet, dass** das Betätigungsglied (7) zur Ausschüttung der eingestellten Produktdosis mittels Drücken relativ zu dem Dosierglied (3) verschiebbar ist.

3. Antriebs- und Dosiervorrichtung nach Anspruch 1, **dadurch gekennzeichnet, dass** die Kupplung (2b, 3c) ein Kupplungsglied (2) aufweist, welches relativ zu dem Dosierglied (3) bei geschlossener Kupplung drehfest ist, so dass das Kupplungsglied (2) eine Drehbewegung eines Rotationsglieds (1) mitmacht, wodurch die Ausschüttfeder (11) gespannt wird und wobei das Kupplungsglied (2) bei geöffneter Kupplung relativ zu dem Dosierglied (3) drehbar ist, und welches drehfest und vorzugsweise axial verschiebbar mit dem Dosisanzeigeelement (10) verbunden ist.

4. Antriebs- und Dosiervorrichtung nach Anspruch 3, **gekennzeichnet durch** ein Ratschenelement (19), welches ein erstes federnd angeordnetes Rastelement (19a) und ein zweites federnd angeordnetes Rastelement (19b) bildet und das mit einem Ende der Ausschüttfeder (11) verbunden ist, wobei das erste Rastelement (19a) in einem Rasteingriff mit dem Kupplungsglied (2) ist, der eine Drehbewegung des Kupplungsglieds in eine Drehrichtung auf das Ratschenelement (19) überträgt und in die entgegengesetzte Drehrichtung nicht überträgt, und wobei das zweite Rastelement (19b) in einem Rasteingriff mit dem Rotationsglied (1) ist, der eine Drehbewegung des Ratschenelements (19) relativ zu dem Rotationsglied (1) in eine Drehrichtung zulässt und in die entgegengesetzte Drehrichtung sperrt.

5. Antriebs- und Dosiervorrichtung nach Anspruch 4, **gekennzeichnet durch** eine Kupplung (le, 9k), welche das Rotationsglied (1) verdrehfest mit dem Gehäuse (4) koppelt, wenn das Betätigungsglied (7) unbetätigt ist, wobei die Kupplung (le, 9k) mittels Betätigung des Betätigungsglieds (7) lösbar ist, so dass das Rotationsglied (1) relativ zu dem Gehäuse (4) drehbar ist.

6. Antriebs- und Dosiervorrichtung nach einem der vorhergehenden Ansprüche, **dadurch gekennzeichnet, dass** die Ausschüttfeder (11) eine wendel- oder spiralförmige Drehfeder ist, wobei ein erstes Ende der Feder (11) fest mit dem Gehäuse (4) und ein zweites Ende der Feder (11) drehfest mit dem Dosisanzeigeelement (10), dem Kupplungsglied (2) nach Anspruch 3, oder dem Ratschenelement (19) nach Anspruch 4 verbunden oder gekoppelt ist.

7. Antriebs- und Dosiervorrichtung nach Anspruch 1, **dadurch gekennzeichnet, dass** das Lagerelement (9) einen federnd angeordneten Schnapper (9i) aufweist, an dem ein Kupplungsglied (2) an einer Anstoßstelle anstößt, wenn das Betätigungsglied (7) für eine Produktausschüttung betätigt ist, wobei der Schnapper (9i) mittels des Dosisanzeigeelements (10) auslenkbar ist, wodurch der Schnapper (9i) an der Anstoßstelle vorbeischiebbar ist.

8. Antriebs- und Dosiervorrichtung nach Anspruch 7, **dadurch gekennzeichnet, dass** auf das Lagerelement (9) eine Rückstellfeder (12b) wirkt, welche den Schnapper (9i) an der Anstoßstelle vorbeischiebt wenn an der Zeigeeinrichtung (4d) ein Dosiswert kleiner als 2 IU ablesbar ist.

9. Antriebs- und Dosiervorrichtung nach Anspruch 1, **dadurch gekennzeichnet, dass** das Lagerelement (9) so mit einem Kupplungsglied (2) in einem Eingriff ist, dass das Kupplungsglied (2) relativ zu dem Lagerelement (9) drehbar und axialfest ist.

10. Antriebs- und Dosiervorrichtung nach einem der vorhergehenden Ansprüche, **dadurch gekennzeichnet, dass** die Ausschüttfeder (11) bei Drehung des Dosierglieds (3) in eine erste Drehrichtung, die eine Dosiserhöhung bewirkt, gespannt wird, und bei Drehung des Dosierglieds (3) in eine zweite Drehrichtung, die eine Dosisverringerung bewirkt, entspannt oder nicht entspannt wird.

11. Antriebs- und Dosiervorrichtung nach Anspruch 1, **dadurch gekennzeichnet dass** eine Betätigung des Betätigungsglieds (7) bewirkt, dass das Lagerelement (9) zusammen mit dem Dosisanzeigeelement (10) relativ zu dem Gehäuse (4) und entlang der Drehachse (L) verschoben wird und/oder dass ein Vortriebsglied (8), dessen distales Ende (8d) vorgesehen ist, auf einen Kolben (14a) eines an der Antriebs- und Dosiervorrichtung befestigten oder befestigbaren Produktbehälters (14) zu wirken, in distale Richtung verschoben wird, und vorzugsweise, dass die Betätigung des Betätigungsglieds (7) ferner bewirkt, dass das Dosisanzeigeelement (10) relativ zu dem Lagerelement (9) gedreht wird in eine Richtung, dass die sich bei der Drehbewegung an der Zeigeeinrichtung (4d) vorbeibewegenden Werte der Dosisskala (10b) zurückzählen.

12. Antriebs- und Dosiervorrichtung nach einem der vorhergehenden Ansprüche, ferner umfassend
- ein Vortriebsglied (8), dessen distales Ende (8d) vorgesehen ist, auf einen Kolben (14a) eines an der Antriebs- und Dosiervorrichtung befestigten oder befestigbaren Produktbehälters (14) zu wirken, wobei das Vortriebsglied (8) ein Gewinde und eine Längsführung aufweist und
- ein Rotationsglied (1), welches in eines aus Gewinde und Längsführung des Vortriebsglieds eingreift, wobei das andere aus Gewinde und Längsführung in einem Eingriff mit dem Gehäuse oder einem gehäusefesten Element ist,
- und eine Kupplung (9k, le), die durch Drücken des Betätigungsglieds (7) die Drehung des Rotationsglieds (1) relativ zu dem Gehäuse (4) freigibt und durch Loslassen des Betätigungsglieds (1) die Drehung des Rotationsglieds (1) relativ zu dem Gehäuse (4) blockiert.

13. Antriebs- und Dosiervorrichtung nach einem der vorhergehenden Ansprüche, **dadurch gekennzeichnet, dass** das Dosisanzeigeelement (10) einen Anschlag (10c) aufweist, der von einem Gegenanschlag (4f) wegbewegt wird, wenn eine Dosiserhöhung vorgenommen wird, und der zu dem Gegenanschlag (4f) hinbewegt wird, wenn eine Dosisverringerung vorgenommen wird oder wenn die Vorrichtung für die Ausschüttung der eingestellten Produktdosis betätigt wird.

14. Antriebs- und Dosiervorrichtung nach einem der vorhergehenden Ansprüche, **dadurch gekennzeichnet, dass** das Dosisanzeigeelement (10) während des Einstellens der Produktdosis, d.h. bei Dosiserhöhung und Dosisverringerung, von dem Rotationsglied (1) zumindest rotatorisch entkoppelt ist und bei der Betätigung der Vorrichtung zum Ausschütten der Produktdosis mit dem Rotationsglied (1) gekoppelt ist.

## Claims

1. A driving and dosing device for an injection device for administering a liquid product,
wherein a product dose to be administered is set with the driving and dosing device, comprising:
a) a housing(4) ,
b) a dose indicating element (10), over the periphery of which a dose scale (10b) is arranged,
c) a pointing device (4d) and a dosing element (3), which can be gripped by the user of the driving and dosing device, wherein by rotating the dosing element (3) relative to the pointing device (4d) in order to set the dose to be administered, the dose indicating element (10) is rotated to move relative to the pointing device (4d) and about an axis of rotation (L), and a value of the dose scale (10b) corresponding to the set dose can be read off by means of the pointing device (4d),
d) a discharge spring (11), which stores the energy necessary for discharging of the product, wherein the discharge spring (11) is cocked by rotating the dosing element (3),
e) an actuating element (7) that is actuated by the user of the driving and dosing device in order to discharge the set product dose,
wherein
f) the driving and dosing device comprises a clutch (2b, 3c) that couples the dosing element (3) to the discharge spring (11) in such a manner that the discharge spring (11) is cocked by rotating the dosing element (3) when the actuating element (7) is not actuated, wherein the clutch (2b, 3c) decouples the dosing element (3) from the discharge spring (11) when the actuating element (7) is actuated,
**characterized by**
a bearing element (9) with which the dose indicating element (10) has an engagement that causes the rotational or screwing movement of the dose indicating element (10) relative to the pointing device (4d), wherein the bearing element (9) is displaceable together with the dose indicating element (10) relative to the housing (4) and along the axis of rotation (L).

2. A driving and dosing device according to claim 1, **characterized in that** the actuating element (7) is displaced by means of pressing relative to the dosing element (3) in order to discharge the set product dose.

3. A driving and dosing device according to claim 1, wherein the clutch (2b, 3c) has a clutch element (2) which is non-rotatable relative to the dosing element (3) when the clutch is engaged such that the clutch element (2) follows a rotational movement of a rotation element (1) whereby the discharge spring (11) is cocked, and wherein the clutch element (2) is rotatable relative to the dosing element (3) when the clutch is disengaged, and that is connected rotationally fixedly and axially displaceably to the dose indicating element (10).

4. A driving and dosing device according to claim 3, further comprising a ratchet element (19) that forms a first resiliently arranged catch element (19a) and a second resiliently arranged catch element (19b) and is connected to one end of the discharge spring (11), wherein the first catch element (19a) has an interlocking engagement with the clutch element (2) that transfers a rotational movement of the clutch element in one rotational direction to the ratchet element (19) and does not transmit it in an opposite rotational direction, and wherein the second catch element (19b) has an interlocking engagement with the rotation element (1) that permits a rotational movement of the ratchet element (19) relative to the rotation element (1) in one rotational direction and blocks it in an opposite rotational direction.

5. A driving and dosing device according to claim 4, further comprising a clutch (1e, 9k) that couples the rotation element (1) secured against torsion to the housing (4) when the actuating element (7) is not actuated, wherein the clutch (1e, 9k) is disengaged by actuating the actuating element (7), so that the rotation element (1) is rotatable relative to the housing.

6. A driving and dosing device according to one of the preceding claims, wherein the discharge spring (11) is a helical or coil spring, wherein a first end of the spring (11) is fixedly connected to the housing (4) and a second end of the spring (11) is rotationally fixedly connected or coupled to the dose indicating element (10), the clutch element (2) of claim 3, or the ratchet element of claim 4.

7. A driving and dosing device according to claim 1, **characterized in that** the bearing element (9) has a resiliently arranged catch (9i), against which a clutch element (2) strikes at a striking point on an end face, when an actuating element (7) is actuated for discharging a product, wherein the catch (9i) is deflected by means of the dose indicating element (10), whereby the catch (9i) is displaced past the striking point axially.

8. A driving and dosing device according to claim 7, **characterized in that** a reset spring (12b) acts on the bearing element (9), pushing the catch (9i) past the striking point, when a dose value less than 2 IU can be read off at the pointing device (4d).

9. A driving and dosing device according to claim 1, **characterized in that** the bearing element (9) is engaged with a clutch element (2) such that the clutch element (2) is rotatable relative to the bearing element (9) and is axially fixed.

10. A driving and dosing device according to one of the preceding claims, wherein the discharge spring (11) is cocked upon rotation of the dosing element (3) in a first rotational direction, which causes a dose increase, and is relaxed or not relaxed upon rotation of the dosing element (3) in a second rotational direction, which causes a reduction of the dose.

11. A driving and dosing device according to claim 1, **characterized in that** actuation of the actuating element (7) has the effect that the bearing element (9) is displaced together with the dose indicating element (10) relative to the housing (4) and along the axis of rotation (L), and/or that a propulsion element (8), the distal end of which is provided to act on a piston (14a) of a product container (14) that is fixed to the driving and dosing device, is displaced in the distal direction, and the actuation of the actuating element (7) has the further effect that the dose indicating element (10) is rotated or screwed, relative to the bearing element (9) in a direction such that the values of the dose scale (10b) passing by the pointing device (4d) as the dose indicating element is rotated or screwed count down.

12. A driving and dosing device according to one of the preceding claims, further comprising
- a propulsion element (8), the distal end (8d) of which is provided to act on a piston (14a) of a product container (14) fixed to the dosing and driving device, wherein the propulsion element (8) has a thread and a longitudinal guide,
- a rotation element (1) that engages with either a thread or a longitudinal guide of the propulsion element, the respective other of the elements consisting of thread and longitudinal guide being engaged with the housing or an element fixed relative to the housing, and
- a clutch (9k, 1e) that enables rotation of the rotation element (1) relative to the housing (4) upon pressing of the actuating element (7) and blocks the rotation of the rotation element (1) relative to the housing (4) upon release of the actuating element (1).

13. A driving and dosing device according to one of the preceding claims, wherein the dose indicating element (10) has a stop (10c) that is moved away from a mating stop (4f) when a dose increase is being performed, and is moved toward the mating stop (4f) when a dose reduction is being performed or when the device is actuated for discharging the set product dose.

14. A driving and dosing device according to one of the preceding claims, wherein the dose indicating element (10) is at least rotationally decoupled from a rotation element (1) during setting of the product dose, i.e. during increasing and decreasing the dose, and is coupled to the rotation element (1) upon actuation of the device for discharging the product dose.

## Revendications

1. Dispositif d'entraînement et de dosage pour un dispositif d'injection pour l'administration d'un produit liquide, dans lequel une dose de produit à administrer peut être réglée avec le dispositif d'entraînement et de dosage, comprenant :
a) un boîtier (4),
b) un élément d'affichage de dose (10) sur la périphérie duquel une échelle de dosage (10b) est agencée,
c) un dispositif de pointage (4d) et un organe de dosage (3) préhensible par l'utilisateur du dispositif d'entraînement et de dosage, dans lequel, pour le réglage de la dose à administrer par rotation de l'organe de dosage (3) par rapport au dispositif de pointage (4d), l'élément d'affichage de dose (10) peut être tourné par rapport au dispositif de pointage (4d) et autour d'un axe de rotation (L), et une valeur de l'échelle de dosage (10b) peut être lue au moyen du dispositif de pointage (4d), laquelle correspond à la dose réglée,
d) un ressort de déversement (11) qui stocke l'énergie nécessaire au déversement du produit, dans lequel le ressort de déversement (11) est tendu ou peut être tendu par rotation de l'organe de dosage (3),
e) un organe d'actionnement (7) actionnable par l'utilisateur du dispositif d'entraînement et de dosage pour le déversement de la dose de produit réglée,
dans lequel
f) le dispositif d'entraînement et de dosage comporte un couplage (2b, 3c) qui raccorde l'organe de dosage (3) au ressort de déversement (11) de sorte que lorsque l'organe d'actionnement n'est pas actionné (7), le ressort de déversement (11) puisse être tendu par rotation de l'organe de dosage (3), et que par actionnement de l'organe d'actionnement (7) l'organe de dosage (3) soit découplé du ressort de déversement (11),
**caractérisé par** un élément de palier (9), avec lequel l'élément d'affichage de dose (10) est en prise, lequel provoque le mouvement de rotation ou de vissage de l'élément d'affichage de dose (10) par rapport au dispositif de pointage (4d), dans lequel l'élément de palier (9) peut être déplacé conjointement avec l'élément d'affichage de dose (10) par rapport au boîtier (4) et le long de l'axe de rotation (L).

2. Dispositif d'entraînement et de dosage selon la revendication 1, **caractérisé en ce que** l'organe d'actionnement (7) peut être déplacé par rapport à l'organe de dosage (3) pour le déversement de la dose de produit réglée par pression.

3. Dispositif d'entraînement et de dosage selon la revendication 1, **caractérisé en ce que** le couplage (2b, 3c) présente un organe de couplage (2) qui est fixe en rotation par rapport à l'organe de dosage (3) lorsque le couplage est fermé, suivant une rotation d'un organe de rotation (1) de manière à ce que le ressort de déversement (11) soit tendu, et qui peut être tourné par rapport à l'organe de dosage (3) lorsque le couplage est ouvert, et qui est raccordé sans pouvoir tourner et de préférence de manière mobile axialement à l'élément d'affichage de dose (10).

4. Dispositif d'entraînement et de dosage selon la revendication 3, **caractérisé par** un élément de rochet (19) qui forme un premier élément d'encliquetage (19a) agencé de manière élastique et un second élément d'encliquetage (19b) agencé de manière élastique et qui est raccordé à une extrémité du ressort de déversement (11), dans lequel le premier élément d'encliquetage (19a) est dans une prise d'encliquetage avec l'organe de couplage (2) qui transmet un mouvement de rotation de l'organe de couplage dans un sens de rotation à l'élément de rochet (19) et ne le transmet pas dans le sens de rotation opposé, et dans lequel le second élément d'encliquetage (19b) est dans une prise d'encliquetage avec l'organe de rotation (1) qui autorise un mouvement de rotation de l'élément de rochet (19) par rapport à l'organe de rotation (1) dans un sens de rotation et le bloque dans le sens de rotation opposé.

5. Dispositif d'entraînement et de dosage selon la revendication 4, **caractérisé par** un couplage (le, 9k) qui couple l'organe de rotation (1) sans pouvoir tourner au boîtier (4) quand l'organe d'actionnement (7) n'est pas actionné, dans lequel le couplage (le, 9k) est détachable par actionnement de l'organe d'actionnement (7) de sorte que l'organe de rotation (1) puisse tourner par rapport au boîtier (4).

6. Dispositif d'entraînement et de dosage selon l'une quelconque des revendications précédentes, **caractérisé en ce que** le ressort de déversement (11) est un ressort de rotation hélicoïdal ou spiralé, dans lequel une première extrémité du ressort (11) est raccordée ou couplée fixement au boîtier (4) et une seconde extrémité du ressort (11) est raccordée ou couplée à l'élément d'affichage de dose (10), à l'organe de couplage (2) selon la revendication 3, ou à l'élément de rochet (19) selon la revendication 4 sans pouvoir tourner.

7. Dispositif d'entraînement et de dosage selon la revendication 1, **caractérisé en ce que** l'élément de palier (9) présente un cliquet (9i) agencé de manière élastique, au niveau duquel un organe de couplage (2) bute contre un point d'aboutement quand l'organe d'actionnement (7) est actionné pour un déversement de produit, dans lequel le cliquet (9i) peut être dévié au moyen de l'élément d'affichage de dose (10), moyennant quoi le cliquet (9i) peut être déplacé au-delà du point d'aboutement.

8. Dispositif d'entraînement et de dosage selon la revendication 7, **caractérisé en ce qu'**un ressort de rappel (12b) agit sur l'élément de palier (9), lequel déplace le cliquet (9i) au-delà du point d'aboutement quand une valeur de dose inférieure à 2 IU peut être lue sur le dispositif de pointage (4d).

9. Dispositif d'entraînement et de dosage selon la revendication 1, **caractérisé en ce que** l'élément de palier (9) est en prise avec un organe de couplage (2) de sorte que l'organe de couplage (2) puisse tourner et soit fixe axialement par rapport à l'élément de palier (9).

10. Dispositif d'entraînement et de dosage selon l'une quelconque des revendications précédentes, **caractérisé en ce que** le ressort de déversement (11) est tendu lors de la rotation de l'organe de dosage (3) dans un premier sens de rotation qui provoque une augmentation de dose, et est détendu ou n'est pas détendu lors de la rotation de l'organe de dosage (3) dans un second sens de rotation qui provoque une diminution de dose.

11. Dispositif d'entraînement et de dosage selon la revendication 1, **caractérisé en ce qu'**un actionnement de l'organe d'actionnement (7) a pour effet que l'élément de palier (9) soit déplacé conjointement avec l'élément d'affichage de dose (10) par rapport au boîtier (4) et le long de l'axe de rotation (L) et/ou qu'un organe d'avancement (8) dont l'extrémité distale (8d) est prévue afin d'agir sur un piston (14a) d'un récipient de produit (14) fixé ou pouvant être fixé sur le dispositif d'entraînement et de dosage, soit déplacé dans le sens distal, et de préférence que l'actionnement de l'organe d'actionnement (7) a de plus pour effet que l'élément d'affichage de dose (10) soit tourné par rapport à l'élément de palier (9) dans un sens dans lequel les valeurs de l'échelle de dosage (10b) se déplaçant devant le dispositif de pointage (4d) lors du mouvement de rotation vont à rebours.

12. Dispositif d'entraînement et de dosage selon l'une quelconque des revendications précédentes, comprenant de plus
- un organe d'avancement (8) dont l'extrémité distale (8d) est prévue pour agir sur un piston (14a) d'un récipient de produit (14) fixé ou pouvant être fixé sur le dispositif d'entraînement et de dosage, dans lequel l'organe d'avancement (8) présente un filet et un guidage longitudinal et
- un organe de rotation (1) qui se met en prise dans un parmi le filet et le guidage longitudinal de l'organe d'avancement, dans lequel l'autre parmi le filet et le guidage longitudinal est en prise avec le boîtier ou un élément fixé au boîtier,
- et un couplage (9k, le) qui libère par pression de l'organe d'actionnement (7) la rotation de l'organe de rotation (1) par rapport au boîtier (4) et bloque par relâchement de l'organe d'actionnement (1) la rotation de l'organe de rotation (1) par rapport au boîtier (4).

13. Dispositif d'entraînement et de dosage selon l'une quelconque des revendications précédentes, **caractérisé en ce que** l'élément d'affichage de dose (10) présente une butée (10c) qui est déplacée à partir d'une contrebutée (4f) quand une augmentation de dose est entreprise, et qui est déplacée vers la contrebutée (4f) quand une diminution de dose est entreprise ou quand le dispositif est actionné pour le déversement de la dose de produit réglée.

14. Dispositif d'entraînement et de dosage selon l'une quelconque des revendications précédentes, **caractérisé en ce que** l'élément d'affichage de dose (10) est découplé de l'organe de rotation (1) au moins par rotation pendant le réglage de la dose de produit, c'est-à-dire cas d' augmentation de dose et de diminution de dose, et est couplé à l'organe de rotation (1) lors de l'actionnement du dispositif pour le déversement de la dose de produit.
